# EUROPEAN PATENT SPECIFICATION

(11) **EP 3 080 283 B1**
(45) Date of publication and mention of the grant of the patent: **22.01.2020**
(21) Application number: 14869980.4
(22) Date of filing: 05.12.2014
(51) Int. Cl.: C12P 7/20, C12N 9/10

(54) **RECOMBINANT STRAIN OF SACCHAROMYCES CEREVISIAE OVERPRODUCING GLYCEROL**
REKOMBINANTER STAMM VON SACCHAROMYCES CEREVISIAE ÜBERPRODUZIERENDEM GLYCERIN
SOUCHE RECOMBINANTE DE SACCHAROMYCES CEREVISIAE SURPRODUCTRICES DE GLYCÉRINE

(30) Priority: 12.12.2013 US 201361915252 P
(43) Date of publication of application: 19.10.2016
(73) Proprietor: Archer Daniels Midland Company, Decatur, Illinois 62526 (US)
(72) Inventor: SIBIRNY, Andriy, Lviv, 79005 (UA); DMYTRUK, Kostyantyn V., Lviv, 79021 (UA); ABBAS, Charles, Champaign, Illinois 61820 (US); MURASHCHENKO, Lidiia R., Lviv, 79005 (UA)
(74) Representative: dompatent von Kreisler Selting Werner - Partnerschaft von Patent- und Rechtsanwälten mbB
(86) International application number: PCT/US2014/068699
(87) International publication number: WO 2015/088892

(56) References cited:
- WO-A2-2011/040901
- WO-A2-2011/040901
- WO-A2-2013/076144
- US-A1- 2010 167 364
- US-B2- 8 232 089
- US-B2- 8 455 239
- DAWID BRAT ET AL: "Cytosolic re-localization and optimization of valine synthesis and catabolism enables inseased isobutanol production with the yeast Saccharomyces cerevisiae", BIOTECHNOLOGY FOR BIOFUELS, BIOMED CENTRAL LTD, GB, vol. 5, no. 1, 6 September 2012 (2012-09-06), page 65, XP021117109, ISSN: 1754-6834, DOI: 10.1186/1754-6834-5-65
- TAXIS ET AL.: 'System of centromeric, episomal, and integrative vectors based on drug resistance markers for Saccharomyces cerevisiae' BIOTECHNIQUES vol. 40, no. 1, 01 January 2006, pages 73 - 78, XP001537899

## Description

### FIELD OF THE INVENTION

The present disclosure relates to modified yeast for enhanced production of glycerol, more particularly to yeast modified to overexpress a truncated *ILV2* gene in *S. cerevisiae,* resulting in an increase in glycerol production under anaerobic growth conditions.

### BACKGROUND OF THE INVENTION

Glycerol (1, 2, 3-propanetriol) is used in the cosmetic, paint, automotive, food, tobacco, pharmaceutical, pulp and paper, leather and textile industries. Glycerol has primarily been recovered as a by-product of biodiesel or soap manufacturing or produced from propylene and allyl alcohol. Alternatively, glycerol has also been produced by microbial fermentation, using carbohydrate-based feedstocks. Glycerol has also been considered as a feedstock for new industrial fermentations. For example, glycerol has been fermented to 1, 3-propanediol, dihydroxyacetone and dihydroxyacetone phosphate and other value-added products.

Due to environmental concerns, the chemical synthesis of glycerol from propylene or allyl alcohol is on the decline. The glycerol obtained as a by-product from biodiesel or soap production contains a lot of impurities, which precludes its further use without additional refining.

Several processes are known for the microbial synthesis of glycerol from carbohydrates that use osmotolerant yeasts, algae and a number of bacteria. However, all these utilize aerobic conditions, so the glycerol production demands air or oxygen purging throughout the fermentation, which considerably increases production costs.

It is known that wild-type strains of *S. cerevisiae* are able to produce substantial amounts of glycerol after adding sulfites to the fermentation medium, which results in trapping of acetaldehyde at an alkaline pH. However, glycerol yield from this sulfite process is typically low, and this mode of production results in large buildup of sulfite, leading to waste stream byproduct disposal problems. The metabolic engineering of *S. cerevisiae* strains with high yield of glycerol that accumulates under anaerobic conditions without buildup of waste byproducts would be much more efficient. However, whereas anaerobic glucose conversion to ethanol is energy positive (net yield of 2 ATP per mole of glucose), anaerobic glycerol production from glucose presents a major challenge as it is an energy-negative process (minus 2 moles of ATP per mole of glucose). This is because glucose first has to be phosphorylated twice, with no production of ATP during glycerol synthesis. This means that yeast cannot survive anaerobically when it accumulates only glycerol, as it needs energy for cell maintenance. To be able to survive, cells either need to get some oxygen for ATP production by oxidative phosphorylation, or need to accumulate some by-product, the synthesis of which is coupled to ATP production. If the cells accumulate equal amounts of glycerol and ethanol, ATP balance is zero and this ratio of glycerol to ethanol can theoretically support strict anaerobic growth. Demonstration of this hypothesis can be demonstrated through the construction of yeast strains through the use of genetic engineering that allow for the production of glycerol and ethanol under anaerobic conditions where the strains can derive sufficient ATP and NADH.

There are a number of publications that describe the increase in glycerol production by *S. cerevisiae* mutants deleted in one or several *ADH* genes coding for alcohol dehydrogenases (Drewke et al., 1990) or *PDC1* and *PDC5* genes coding for pyruvate decarboxylases (Nevoigt and Stahl, 1996). Mutants defective in alcohol dehydrogenase typically accumulate large amounts of acetaldehyde and acetic acid which are toxic to yeast cells. Mutants defective in pyruvate decarboxylase look more promising. However, they cannot grow without adding exogenous ethanol or acetate, as pyruvate decarboxylase is the only source of two-carbon compounds in cytosol that are needed mainly for lipid biosynthesis (Pronk et al., 1996). The largest amount of glycerol is accumulated by *S. cerevisiae* mutants deleted in the *TPI1* gene coding for triose phosphate isomerase (Overkamp et al., 2002). Unfortunately, such mutants fail to grow on glucose as the primary carbon source. Metabolic engineering of an alternative NADH reoxidation pathway (isocitrate lyase) has allowed partial restoration of the growth on glucose, however the robustness of strains was low. Additionally, glucose inhibited growth of the analyzed *TPI1* strains.

Therefore, there is continuing need in the field to develop yeast that are capable of efficient glycerol production from glucose under anaerobic conditions. Brat et al,. Biotechnology for Biofuels, 2012, 5, page 65 discloses the re-location of Ilv2 in S. cerevisiae from the mitochondrial matrix into the cytosol by deleting 54 amino acids from the N-terminus under a truncated HXT7 promoter fragment. WO 2011/040901 discloses a high flux in conversion of pyruvate to cetolactate achieved in yeast through expression of acetolactate synthase in cytosol in conjunction with reduction in pyruvate decarboxylase activity.

### BRIEF SUMMARY OF THE INVENTION

The present disclosure provides methods for increasing glycerol production by increasing expression of acetolactate synthase in recombinant yeast and genetic tools for producing said recombinant yeasts. One embodiment of the method of the invention involves a yeast comprising a recombinant nucleic acid with a truncated portion of a gene encoding a cytosol located acetolactate synthase activity, wherein said gene is operably linked to a non-native promoter to express said acetolactate synthase activity in the cytosol and wherein said truncated gene does not comprise a mitochondrial targeting signal.

Further disclosed herein is a nucleic acid molecule with a gene encoding the protein of a cytosol located acetolactate synthase, wherein said gene is at least 75% identical to SEQ ID NO:2 and does not contain a mitochondrial targeting signal.

A further embodiment of the method of the invention includes the recombinant nucleic acid molecule with the cytosol located acetolactate synthase activity gene being SEQ ID NO: 1.

One embodiment of the method of the invention contains the recombinant nucleic acid molecule with an *ADH1* promoter operably linked to the truncated ILV2 gene. Further disclosed herein is a vector containing the truncated ILV2 gene operably linked to a strong constitutive promoter. Additionally, the vector may include a selective marker. The selective marker of the vector may be a natNT2 gene.

Further disclosed herein is a host cell including a vector containing the truncated ILV2 gene operably linked to a strong constructive promoter. Additionally, the host cell may comprise the vector containing the truncated ILV2 gene operably linked to a strong constructive promoter and including a selective marker. The host cell may be a *S*. *cerevisiae* cell.

One embodiment of the method of the invention involves a yeast strain containing a recombinant nucleic acid molecule comprising a truncated portion of a gene encoding a cytosol located acetolactate synthase activity that is operably linked to a non-native promoter to express the acetolactate synthase activity in the cytosol and wherein said truncated gene does not comprise a mitochondrial targeting signal.

A further embodiment includes the use of a yeast strain comprising a truncated portion of a gene encoding a cytosol located acetolactate synthase activity that is operably linked to a non-native promoter to express the acetolactate synthase activity in the cytosol when the truncated gene is according to SEQ ID NO: 1. A further embodiment is when the promoter element of this yeast strain is an *ADH1* promoter. An even further embodiment is when this yeast strain is *S. cerevisiae.*

One embodiment of the present invention includes a method for enhancing glycerol production in yeast comprising growing the yeast strain comprising a recombinant nucleic acid molecule comprising a truncated portion of a gene encoding a cytosol located acetolactate synthase activity that is operably linked to a non-native promoter to express the acetolactate synthase activity in the cytosol, wherein said truncated gene does not comprise a mitochondrial targeting signal, anaerobically in a culture medium, under conditions that cause the yeast strain to make glycerol.

A further embodiment includes a method of growing the yeast strain comprising a recombinant nucleic acid molecule comprising a truncated portion of a gene encoding a cytosol located acetolactate synthase activity that is operably linked to a non-native promoter to express the acetolactate synthase activity in the cytosol, wherein said truncated gene does not comprise a mitochondrial targeting signal, anaerobically in a culture medium, under conditions that cause the yeast strain to make glycerol, wherein an amount of glycerol produced by said yeast strain is more than four times greater than an amount of glycerol produced by a corresponding yeast strain that does not contain said recombinant nucleic acid molecule but is otherwise identical to said yeast strain.

A further embodiment includes the yeast strain to be *S. cerevisiae.*

### BRIEF DESCRIPTION OF THE DRAWINGS

FIG. 1 shows a linear scheme of recombinant plasmid pUC57-ADHI-ILV2-natNT2 (A) and a linear scheme of recombinant plasmid pUC57-natNT2 -TPI1-ILV2n (B).
FIG. 2 shows the complete sequence of recombinant plasmid pUC57-ADHI-ILV2-natNT2. Components of complete sequence are represented by font style and bold differences, as follows: pUC57 in Calibri 11 (CGGAT), ADHI in Calibri 8 (CGGAT), ILV2 in Calibri 11 Bold (**CGGAT**), natNT2 in Tahoma 12 Italic (*CGGAT*)*,* loxP in Tahoma 12 Bold **(CGGAT)**
FIG. 3 shows the complete sequence of recombinant plasmid pUC57-natNT2-TPI1-ILV2n. Components of complete sequences are represented by font style and bold differences as follows: pUC57 in Calibri 11 (CGGAT), TPI1 in Calibri 8 (CGGAT); ILV2 in Calibri 11 Bold (**CGGAT**), natNT2 in Tahoma 12 Italic (*CGGAT*).

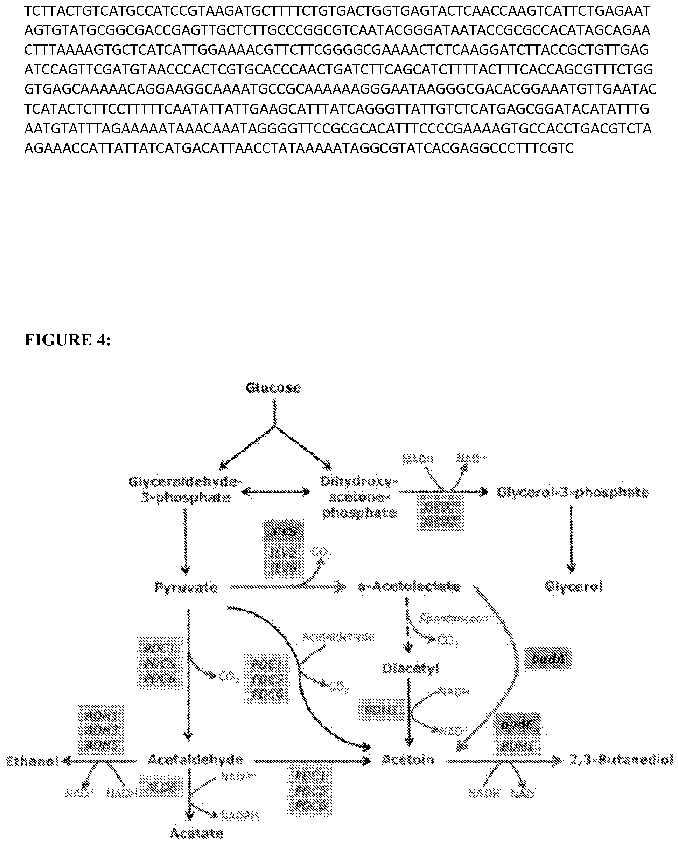

FIG. 4 shows yeast metabolism of glucose and catalytic action of acetolactate synthase to yield glycerol and ethanol.

### SEQUENCE LISTING

SEQ ID NO: 1 - The nucleic acid sequence of the truncated ilV2 gene from *S. cerevisiae.*
SEQ ID NO: 2 - The amino acid sequence of the truncated ilV2 gene from *S. cerevisiae.*
SEQ ID NO: 3 - The nucleic acid sequence of the full length native ilV2 gene from *S. cerevisiae.*
SEQ ID NO: 4 - The amino acid sequence of the full length native ilV2 gene from *S. cerevisiae.*
SEQ ID NO: 5 - The nucleic acid sequence of the ADH1 promoter
SEQ ID NO: 6 - The nucleic acid sequence of the TPI1 promoter
SEQ ID NO: 7 - The nucleic acid sequence of the natNT2 marker
SEQ ID NO: 8 - The nucleic acid sequence of loxP, used to facilitate marker rescue
SEQ ID NO: 9 - The nucleic acid sequence of pUC57-ADHI-ILV2-natNT2
SEQ ID NO: 10 - The nucleic acid sequence of pUC57-natNT2 -TPI1-ILV2n
SEQ ID NO: 11 - Ko574 Primer used to PCR amplify truncated ILV2
SEQ ID NO: 12 - Ko575 Primer used to PCR amplify truncated ILV2
SEQ ID NO: 13 - Ko572 Primer used to PCR amplify ADH1 promoter
SEQ ID NO: 14 - Ko573 Primer used to PCR amplify ADH1 promoter
SEQ ID NO: 15 - OK19 Primer used to PCR amplify natNT2 marker for cloning into plasmid pUC57-ADHI-ILV2-natNT2
SEQ ID NO: 16 - OK20 Primer used to PCR amplify natNT2 marker for cloning into plasmid pUC57-ADHI-ILV2-natNT2
SEQ ID NO: 17 - LY7 Primer used to PCR amplify TPI1 promoter
SEQ ID NO: 18 - LY8 Primer used to PCR amplify TPI1 promoter
SEQ ID NO: 19 - LY9 primer to amplify the full length native ilV2 gene from *S. cerevisiae.*
SEQ ID NO: 20 - LY10 primer to amplify the full length native ilV2 gene from *S. cerevisiae.*
SEQ ID NO: 21 - Ko446 Primer used to amplify natNT2 for cloning into pUC57 natNT2 -TPI1-ILV2n
SEQ ID NO: 22 - Ko448 Primer used to amplify natNT2 for cloning into pUC57 natNT2 -TPI1-ILV2n
SEQ ID NO: 23 - full length ILV2 gene from *Saccharomyces kudriavzevil* (The truncated version of this gene (amino acid 56- 687) has 94.5 % homology to the truncated ILV2 from *S. cerevisiae=* SEQ ID NO: 2.)
SEQ ID NO: 24 - full length ILV2 gene from *Naumovozyma castellii* (The truncated version of this gene (amino acid 52- 684) has 85.2 % homology to the truncated ILV2 from *S. cerevisiae=* SEQ ID NO: 2.)
SEQ ID NO: 25 - full length ILV2 gene from *Naumovozyma dairenensis* (The truncated version of this gene (amino acid 54- 685) has 83.4 % homology to the truncated ILV2 from *S. cerevisiae=* SEQ ID NO: 2.)
SEQ ID NO: 26 - full length ILV2 gene from *Candida glabrata* (The truncated version of this gene (amino acid 41- 677) has 82.8 % homology to the truncated ILV2 from *S. cerevisiae=* SEQ ID NO: 2.)
SEQ ID NO: 27 - full length ILV2 gene from *Torulaspora delbrueckii* (The truncated version of this gene (amino acid 53- 682) has 80.9 % homology to the truncated ILV2 from *S. cerevisiae=* SEQ ID NO: 2.)
SEQ ID NO: 28 - full length ILV2 gene from *Kazachstania africana* (The truncated version of this gene (amino acid 51- 682) has 80.8 % homology to the truncated ILV2 from *S. cerevisiae=* SEQ ID NO: 2.)
SEQ ID NO: 29 - full length ILV2 gene from *Kazachstania naganishii* (The truncated version of this gene (amino acid 53- 693) has 80.6 % homology to the truncated ILV2 from *S. cerevisiae=* SEQ ID NO: 2.)
SEQ ID NO: 30 - full length ILV2 gene from *Vanderwaltozyma polyspora* (The truncated version of this gene (amino acid 46- 671) has 79.8 % homology to the truncated ILV2 from *S. cerevisiae=* SEQ ID NO: 2.)
SEQ ID NO: 31 - full length ILV2 gene from *Zygosaccharomyces rouxii* (The truncated version of this gene (amino acid 72- 700) has 79.2 % homology to the truncated ILV2 from *S. cerevisiae=* SEQ ID NO: 2.)
SEQ ID NO: 32 - full length ILV2 gene from *Zygosaccharomyces bailii* (The truncated version of this gene (amino acid 55- 685) has 78.5 % homology to the truncated ILV2 from *S. cerevisiae=* SEQ ID NO: 2.)
SEQ ID NO: 33 - full length ILV2 gene from *Kluyveromyces lactis* (The truncated version of this gene (amino acid 46- 691) has 77.7 % homology to the truncated ILV2 from *S. cerevisiae=* SEQ ID NO: 2.)
SEQ ID NO: 34 - full length ILV2 gene from *Kluyveromyces marxianus* (The truncated version of this gene (amino acid 68- 700) has 77.25% homology to the truncated ILV2 from *S. cerevisiae=* SEQ ID NO: 2.)
SEQ ID NO: 35 - full length ILV2 gene from *Lachancea thermotolerans* (The truncated version of this gene (amino acid 49- 678) has 76.8 % homology to the truncated ILV2 from *S. cerevisiae=* SEQ ID NO: 2.)
SEQ ID NO: 36 - full length ILV2 gene from *Tetrapisispora phaffia* (The truncated version of this gene (amino acid 53- 688) has 76.6 % homology to the truncated ILV2 from *S. cerevisiae=* SEQ ID NO: 2.)
SEQ ID NO: 37 - full length ILV2 gene from *Tetrapisispora blattae* (The truncated version of this gene (amino acid 79- 710) has 75.4 % homology to the truncated ILV2 from *S. cerevisiae=* SEQ ID NO: 2.)

### DETAILED DESCRIPTION OF PREFERRED EMBODIMENTS

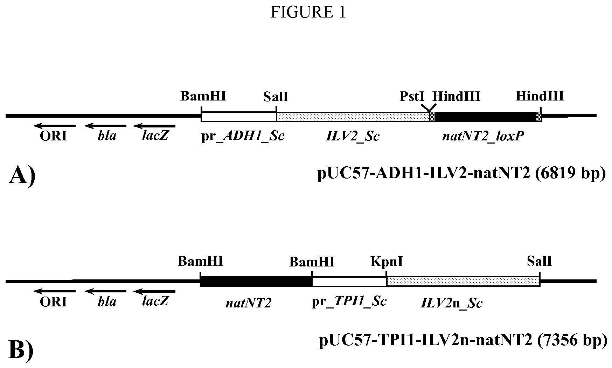

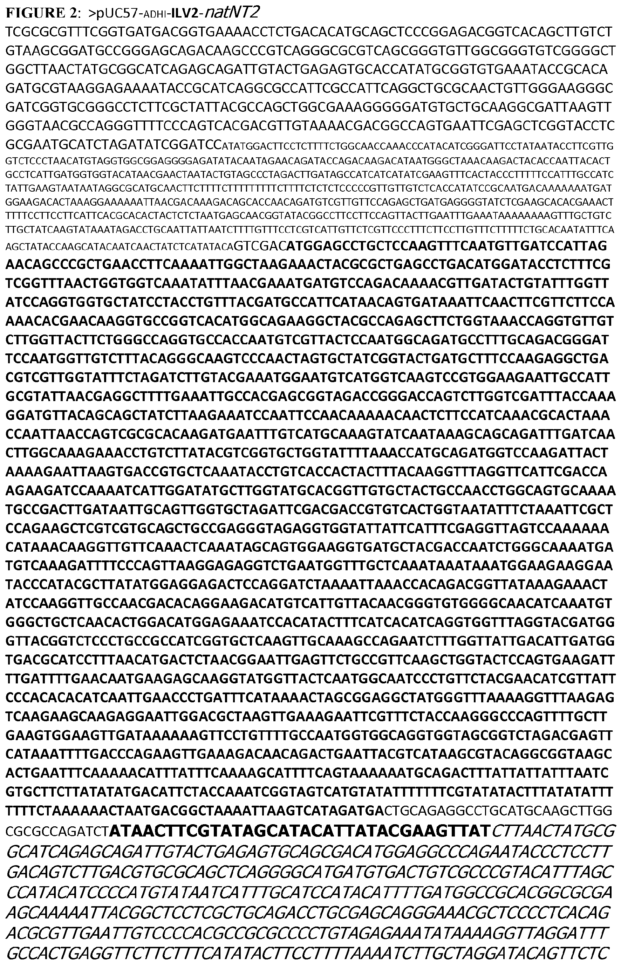

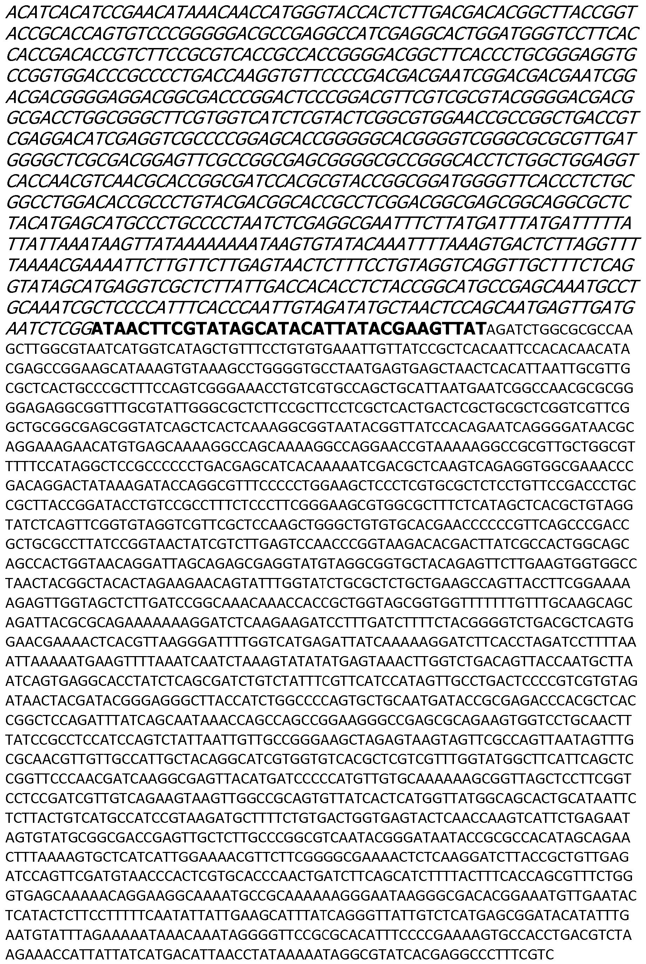

It has been discovered that overexpression of the truncated version (deficient 5'-165 bp and lacking a mitochondrial targeting signal) (SEQ ID NO: 1) of the yeast *ILV2* gene, encoding for acetolactate synthase, strongly activates glycerol production under anaerobic conditions. One aspect of the present invention is directed to using a recombinant nucleic acid molecule formed by fusing the truncated *ILV2* gene with a strong constitutive promoter element. The source of the ILV2 gene could be selected from a eukaryotic microorganism. One suitable promoter element is the promoter of gene *ADH1* encoding alcohol dehydrogenase. The truncated *ILV2* gene, operably linked to the promoter element, is cloned into a vector. Plasmid pUC57 is one suitable vector. Preferably a selective marker is also cloned into the plasmid. Dominant selective marker natNT2, conferring resistance to antibiotic nourseothricin (NTC) for selection in yeast, is one suitable selection marker. An example of a suitable recombinant plasmid is pUC57-ADHI-ILV2-natNT2 (SEQ ID NO: 9) (FIG 1 (A): FIG 2 shows the DNA sequence) harboring an expression cassette for overexpression of the truncated version of the *S. cerevisiae* ILV2 gene.

The recombinant nucleic acid molecule comprising the truncated *ILV2* gene, operably linked to a promoter, is transformed into a host cell. It is preferred that a *Saccharomyces cerevisiae* strain is used as the host strain, more preferably, *S. cerevisiae* strain BY4742.

As discussed in more detail below, the recombinant strain BY4742/ILV2 of *S. cerevisiae,* when grown anaerobically in a suitable medium, under conditions that cause the yeast strain to make glycerol, was shown to produce as much as 4 g of glycerol /l, which was a 4.4-fold improvement in glycerol production as compared to parental strain BY4742.

Preferably, a nucleic acid molecule encoding the truncated *ILV2* gene encoding a protein sequence would be at least 75 %, 76 %, 77 %, 78 %, 79 %, 80 %, 81 %, 82 %, 83 %, 84 %, 85 %, 86 %, 87 %, 88 %, 89 %, 90 %, 91 %, 92 %, 93 %, 94 %, 95 %, 96 %, 97 %, 98 %, or 99 % identical to SEQ ID NO: 2.

The recombinant strain BY4742/ILV2 of *S. cerevisiae* expresses a presumably cytosolic form of *ILV2.* While not wanting to be bound by any particular mode of increased glycerol production, the following is offered as a possible mode. The acetolactate synthase encoded by *ILV2* is normally transported into the mitochondria where it is sequestered from the cytosolic pool of NAD(H) and pyruvate that is used during anaerobic fermentation. The activity of the enzyme is to catalyze the condensation of two pyruvate molecules into an acetolactate molecule with release of carbon dioxide. We suggest that with the transit peptide removed, the enzyme activity is retained in the cytosol where it reduces the available pyruvate pool that ordinarily would proceed predominantly to ethanol and acetate production under anaerobic conditions. The last step in ethanol production is the conversion of acetaldehyde to ethanol by alcohol dehydrogenase with oxidation of NADH to NAD+, which depletes the NADH pool. Because less pyruvate is available for conversion to ethanol, less cytosolic NADH is consumed thereby allowing more of the NADH to be used upstream by NADH dependent glycerol 3-phosphate dehydrogenase, which in turns leads to increased production of glycerol-3-phosphate and its subsequent dephosphoryalation to glycerol (FIG. 2). Unlike the case where ADH activity is completely eliminated, there is still reduction in toxic levels of acetaldehyde produced by the action of alcohol dehydgrogenase. Since pyruvate decarboxylase is not eliminated, there still remains adequate production of cytosolic acetate from pyruvate, with the acetate being necessary for fatty acid synthesis. Accordingly, the increased expression of cytosolic acetolactate synthase is believed to reduce, but not eliminate, the cytosolic pool of pyruvate, leaving sufficient levels of this important metabolic intermediate to maintain fatty acid synthesis. This also permits increased flow of the phosphorylated 3 carbon metabolite into glycerol production due to higher NADH availability because not as much pyruvate is being shunted to ethanol via alcohol dehydrogenase.

### A. DEFINITIONS

The term "gene" refers to a DNA sequence that comprises coding sequences and optionally control sequences necessary for the production of a polypeptide from the DNA sequence.

The term "recombinant DNA" molecule means a hybrid DNA sequence comprising at least two nucleotide sequences not normally found together in nature.

The term "vector" is used in reference to nucleic acid molecules into which fragments of DNA may be inserted or cloned and can be used to transfer DNA segments into a cell and capable of replication in a cell. Vectors may be derived from plasmids, bacteriophages, viruses and cosmids.

The terms "recombinant vector", "expression vector" or "construct" as used herein refer to DNA or RNA sequences containing a desired coding sequence and appropriate DNA or RNA sequences necessary for the expression of the operably linked coding sequence in a particular host organism. Prokaryotic expression vectors include a promoter, a ribosome binding site, an origin of replication for autonomous replication in a host cell and possibly other sequences, e.g. an optional operator sequence, optional restriction enzyme sites. A promoter is defined as a DNA sequence that directs RNA polymerase to bind to DNA and to initiate RNA synthesis. Eukaryotic expression vectors include a promoter, optionally a polyadenylation signal and optionally an enhancer sequence.

A polynucleotide having a nucleotide sequence "encoding a peptide, protein or polypeptide" means a nucleic acid sequence comprising a coding region for the peptide, protein or polypeptide. The coding region may be present in either a cDNA, genomic DNA or RNA form. When present in a DNA form, the oligonucleotide may be single-stranded (i.e., the sense strand) or double-stranded. Suitable control elements such as enhancers/promoters, splice junctions and polyadenylation signals may be placed in close proximity to the coding region of the gene if needed to permit proper initiation of transcription and/or correct processing of the primary RNA transcript. Alternatively, the coding region, utilized in the expression vectors of the present invention may contain endogenous enhancers/promoters, splice junctions, intervening sequences and polyadenylation signals. Further, the coding region may contain a combination of both endogenous and exogenous control elements.

Promoters and enhancers consist of short arrays of DNA sequences that interact specifically with cellular proteins involved in transcription. Promoter and enhancer elements have been isolated from a variety of eukaryotic sources including genes from yeast, insect and mammalian cells. Promoter and enhancer elements have also been isolated from viruses and analogous control elements, such as promoters, are also found in prokaryotes. The selection of a particular promoter and enhancer depends on the cell type used to express the protein of interest. The enhancer/promoter may be "endogenous" or "exogenous" or "heterologous." An "endogenous" enhancer/promoter is one that is naturally linked with a given gene in the genome. An "exogenous" or "heterologous" enhancer/promoter is one that is placed in juxtaposition to a gene by means of genetic manipulation (i.e., molecular biological techniques) such that transcription of the gene is directed by the linked enhancer/promoter. A "constitutive promoter" is an unregulated promoter that allows for continual transcription of its associated gene.

The term "expression system" refers to any assay or system for determining (e.g., detecting) the expression of a gene of interest. Those skilled in the field of molecular biology will understand that any of a wide variety of expression systems may be used.

The term "recombinant protein" or "recombinant polypeptide" as used herein refers to a protein molecule expressed from a recombinant DNA molecule. In contrast, the term "native protein" is used herein to indicate a protein isolated from a naturally occurring (i.e., a non-recombinant or wild type) source. Molecular biological techniques may be used to produce a recombinant form of a protein with identical properties as compared to the native form of the protein.

The terms "cell," "cell line," "host cell," as used herein, are used interchangeably, and all such designations include progeny or potential progeny of these designations. By "transformed cell" is meant a cell into which (or into an ancestor of which) has been introduced a nucleic acid molecule of the invention. Optionally, a nucleic acid molecule disclosed herein may be introduced into a suitable cell line so as to create a stably transfected cell line capable of producing the protein or polypeptide encoded by the nucleic acid molecule. Vectors, cells, and methods for constructing such cell lines are well known in the art. The words "transformants" or "transformed cells" include the primary transformed cells derived from the originally transformed cell without regard to the number of transfers. All progeny may not be precisely identical in DNA content, due to deliberate or inadvertent mutations. Nonetheless, mutant progeny that have the same functionality as screened for in the originally transformed cell are included in the definition of transformants.

The term "operably linked" as used, herein refers to the linkage of nucleic acid sequences in such a manner that a nucleic acid molecule capable of directing the transcription of a given gene and/or the synthesis of a desired protein molecule is produced. The term also refers to the linkage of sequences encoding amino acids in such a manner that a functional (e.g., enzymatically active, capable of binding to a binding partner, capable of inhibiting) protein of polypeptide, or a precursor thereof, e.g., the pre- or prepro-form of the protein or polypeptide, is produced.

### B. MATERIALS AND METHODS USED FOR EXEMPLARY EMBODIMENTS

### Strains.

The *S. cerevisiae* strain BY4742 (MAT.alpha., his3.DELTA1, leu2.DELTA.0, lys2.DELTA.0, ura3.DELTA.0; Giaever et al., 2002) was used for overexpression of the truncated version of the *ILV2* gene. *Escherichia coli* DH5α strain (Φ80d*lac*ZΔM15, *rec*A1*, endA*1*, gyrA96, thi-1, hsdR*17(r_{K}⁻, m_{K}⁺), *supE*44, *relA*1*, deoR, Δ*(*lacZYA-argF*)U169 was used for subcloning.

### DNA Manipulation

Genomic DNA from *S. cerevisiae* was isolated using Wizard® Genomic DNA Purification Kit (Promega, Madison, WI, USA). Plasmid DNA from *E. coli* was isolated using Wizard® Plus SV Minipreps DNA Purification System (Promega) and High Fidelity PCR Enzyme Mix and restriction enzymes were used according to recommendation of supplier (Thermo scientific, Vilnius, Lithuania, EU). *S. cerevisiae* transformation was performed by Sambrook and Russell 2001.

### Construction of Plasmid for Overexpression ILV2

Construction of the expression cassette pUC57-ADHI-ILV2-natNT2 for overexpression of *S. cerevisiae* gene *ILV2* encoding acetolactate synthase, lacking a mitochondrial targeting signal, was performed using the *ILV2* gene deficient in 5'-165 bp (SEQ ID NO: 1) and a strong constitutive promoter of gene *ADH1* encoding alcohol dehydrogenase. The truncated *ILV2* gene and promoter were PCR-amplified using pairs of primers Ko574 (CAA TCA ACT ATC TCA TAT ACA GTC GAC ATG GAG CCT GCT CCA AGT TTC AA) (SEQ ID NO: 11) / Ko575 (AAA CTG CAG TCA TCT ATG ACT TAA TTT TAG CC) (SEQ ID NO: 12) and Ko572 (CGC GGA TCC ATA TGG ACT TCC TCT TTT CTG) (SEQ ID NO: 13) and Ko573 (TTG AAA CTT GGA GCA GGC TCC ATG TCG ACT GTA TAT GAG ATA GTT GAT TG) (SEQ ID NO: 14). Both fragments were combined via overlap PCR using primers Ko575 and Ko572. The fused DNA fragment harboring *ADH1* promoter and truncated version of *ILV2* gene was BamHI/PstI double digested and cloned to the BamHI/PstI linearized pUC57 plasmid cloning vector. The resulting plasmid was designated as pUC57-ADHI-ILV2.

A second marker gene *natNT2,* from *Streptomyces noursei* which provides for resistance to the antibiotic nourseothricin, was PCR-amplified using primer pair OK19 (CCC AAG CTT GGC GCG CCA GAT CTA TAA CTT CGT ATA GCA TAC ATT ATA CGA AGT TAT CTT AAC TAT GCG GCA TCA GAG) (SEQ ID NO: 15) and OK20 (CCC AAG CTT GGC GCG CCA GAT CTA TAA CTT CGT ATA ATG TAT GCT ATA CGA AGT TAT CCG AGA TTC ATC AAC TCA TTG C) (SEQ ID NO: 16), and used with plasmid pRS41N (Taxis and Knop 2006) as a template.

This 1348 bp DNA fragment was HindIII digested and cloned to HindIII-linearized plasmid pUC57-ADHI-ILV2. The plasmid was transformed into *E. coli* as described above, and selection of *E. coli* transformants was performed on LB medium supplemented with nourseothricin at a concentration of 50 µg/mL. The constructed and verified plasmid was designated as pUC57-ADHI-ILV2-natNT2 (SEQ ID NO:9).

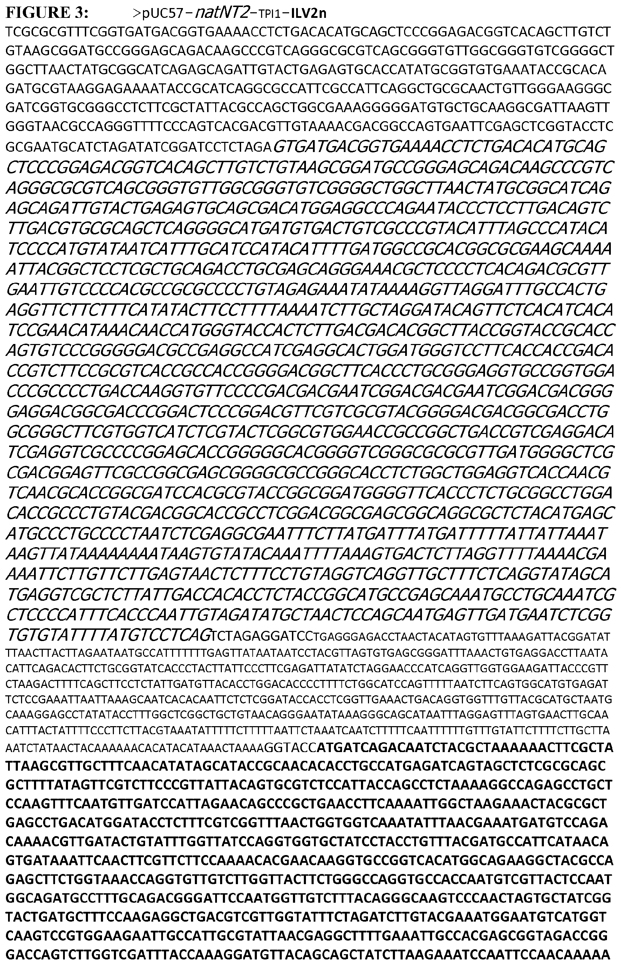

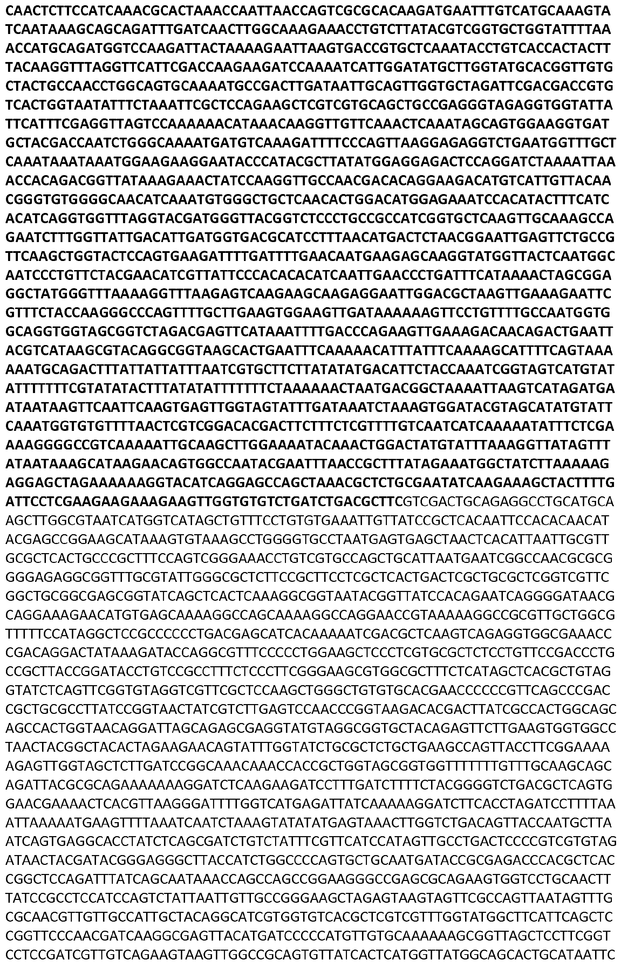

In order to overexpress the native *ILV2* gene coding for the acetolactate synthase, this target gene was fused with the strong constitutive promoter of gene *TPI1* encoding triose phosphate isomerase. The native mitochondrial Ilv2 protein catalyzes the first common step in isoleucine and valine biosynthesis pathway. The *TPI1* promoter and ORF of *ILV2* of *S. cerevisiae* in part with terminator sequence were amplified using pairs of primers LY7 (CGG GAT CCT GAG GGA GAC CTA ACT ACA TAG) (SEQ ID NO: 17) / LY8 (TAG CGT AGA TTG TCT GAT CAT GGT ACC TTT TAG TTT ATG TAT GTG TTT TTT) (SEQ ID NO:18) and LY9 (AAA AAA CAC ATA CAT AAA CTA AAA GGT ACC ATG ATC AGA CAA TCT ACG CTA) (SEQ ID NO: 19)/ LY10 (GCG TCG ACG AAG CGT CAG ATC AGA CAC A) (SEQ ID NO: 20) . Both fragments were combined using primers LY7 / LY10 by overleap PCR and subsequently cloned to the BamHI/SalI double-digested pUC57 plasmid. This plasmid was designated pUC57-TPII-ILV2n. The gene *natNT2* conferring resistance against the aminoglycoside antibiotic nourseothricin was amplified using primers Ko446 (CCG GGA TCC TCT AGA GTG ATG ACG GTG AAA ACC TCT G) (SEQ ID NO: 21)/ Ko448 (CCG GGA TCC TCT AGA CTG AGG ACA TAA AAT ACA CAC CG) (SEQ ID NO: 22) and plasmid pRS41N as a template. The amplified fragment was digested with BamHI and ligated with BamHI digested and dephosphorylated plasmids pUC57-TPI1-ILV2n. The selection of *E. coli* transformants was performed as described above. Constructed and verified plasmid was designated as pUC57-natNT2-TPI1-ILV2n (FIG. 1 (B), DNA sequence in FIG 3).

### Transformation, Selection and Characterization of Stable Transformants of S. cerevisiae

The constructed plasmids pUC57-ADHI-ILV2-natNT2 and pUC57-natNT2-TPI1-ILV2n were BamHI- and SalI-linearized and used to transform the S. *cerevisiae* BY4742 parental strain as described above. The transformants were selected on a solid YPD medium supplemented with nourseothricin (100 µg/ml). The selected transformants were stabilized by alternating cultivation in a non-selective media for 12-14 generations followed by shifting to a selective media containing nourseothricin. The expression system for detecting the presence of the desired plasmid construct in the genome of the stable transformants was confirmed by diagnostic PCR.

### EXAMPLES

The following examples are intended to guide those skilled in the art in the practice of this invention. Example not covered by the scope of the claims are for illustrative purposes.

### Example 1 - Glycerol Overproduction in Transformed S. cerevisiae

The selected *S. cerevisiae* transformants BY4742/ILV2 and BY4742/ILV2n expressing truncated and native form of *ILV2* gene, respectively, and the parental strain BY4742 as a control, were each incubated in separate fermentation broths comprising yeast extract 5 g/l, peptone 10 g/l, D-glucose 50 g/l. Fermentation was carried out at a temperature of 30 °C with limited aeration using a rotary shaker at a setting of 120 revolutions/min. An initial biomass concentration of 1 g/l was used for fermentation. On the second day of fermentation, the recombinant strain BY4742/ILV2 possessed approximately 10 % growth retardation as compared to parental strain BY4742 (Table 1). The recombinant strain BY4742/ILV2 produced more than a 4-fold increase of glycerol production as compared to the parental strain, reaching 4 g of glycerol /l (Table 1). Ethanol synthesis of the recombinant strain BY4742/ILV2 was 1.8-fold reduced as compared to the parental strain (Table 1). During fermentation, biomass accumulation of strain BY4742/ILV2n was similar to that of parental strain. Strain BY4742/ILV2n produced a slight increase in the amount of glycerol (1.1 g/l) when compared to parental strain BY4742 (0.9 g/l). Ethanol synthesis of the strain was 1.3-fold reduced reaching 13 g/l (Table 1). Glucose consumption and acetate production were approximately on the same level for all analyzed strains. To prove overexpression of both versions of *ILV2* gene the specific activity of Ilv2 of the constructed strains was assayed. Specific activities of Ilv2 for strains BY4742/ILV2 and BY4742/ILV2n were increased on 46 and 59 %, respectively, as compared to that of parental strain (Table 1).

**TABLE 1. Biomass, glycerol, ethanol and acetic acid synthesis, glucose consumption, specific activity of Ilv2 of S. cerevisiae strains on the second day of 5 % glucose fermentation.**

| **Strains** | **Biomass (g/l)** | **Analite (g/l)** | | | | **Ilv2 specific activity (U/mg of protein)** |
|---|---|---|---|---|---|---|
| | | **Glucose** | **Acetic acid** | **Ethanol** | **Glycerol** | |
| **BY4742** | 2.25 | 0.5 | 0.3 | 17.1 | 0.9 | 74.4 |
| **BY4742/ILV2** | 2.01 | 0.6 | 0.4 | 9.4 | 4.0 | 108.4 |
| **BY4742/ILV2n** | 2.21 | 0.3 | 0.4 | 13.0 | 1.1 | 118.0 |

### Further Transformed Yeast Overproducing Glycerol

*S. cerevisiae* strains may be engineered using in part the *ILV2* gene with other genes that are involved the synthesis and degradation of glycerol to construct robust *S. cerevisiae* strains that are capable of effective glycerol production from glucose under anaerobic conditions. Strains expressing mitochondrial (native) and cytosolic *ILV2* may be generated on the background of industrial ethanol producing strain AS400. A cytosolic form of *ILV2* may be expressed in strains with altered activity of Pdc (pyruvate decarboxylase), Tpi (triosephosphate isomerase) or Adh (alcohol dehydrogenase) and their combinations.

### REFERENCES

The present application cites several references, summarized herein below. Each such citation is to aid one of ordinary skill in the art in better understanding the present invention and to find sources of sequences, recombinant techniques, tests and other routine information that would enable one of ordinary skill to practice any of numerous embodiments of the present invention.
Wang, Z., Zhuge, J., Fang, H., Prior, B., (2001) Glycerol production by microbial fermentation: A review; Biotechnology Advances 19:201-223.
Drewke, C., Thielen, J., Ciriacy, M. (1990) Ethanol formation in adh0 mutants reveals the existence of a novel acetaldehyde-reducing activity in Saccharomyces cerevisiae; J Bacteriol. 172(7):3909-3917.
Nevoigt E, Stahl U., (1996) Reduced pyruvate decarboxylase and increased glycerol-3-phosphate dehydrogenase [NAD+] levels enhance glycerol production in Saccharomyces cerevisiae. Yeast 12:1331-1337.
Pronk, J., Steensma, H, Van Dijken, J. (1996) Pyruvate Metabolism in Saccharomyces cerevisiae; Yeast 12:1607-1633.
Overkamp, K., Bakker, B., Kötter, P., Luttik, M., Van Dijken, J., Pronk, J. (2002) Metabolic engineering of glycerol production in Saccharomyces cerevisiae; Appl Environ Microbiol. 68(6):2814-21.
Giaever G, Chu AM, Ni L, Connelly C, Riles L, Véronneau S, Dow S, Lucau-Danila A, Anderson K, André B, Arkin AP, Astromoff A, El-Bakkoury M, Bangham R, Benito R, Brachat S, Campanaro S, Curtiss M, Davis K, Deutschbauer A, Entian KD, Flaherty P, Foury F, Garfinkel DJ, Gerstein M, Gotte D, Güldener U, Hegemann JH, Hempel S, Herman Z, Jaramillo DF, Kelly DE, Kelly SL, Kötter P, LaBonte D, Lamb DC, Lan N, Liang H, Liao H, Liu L, Luo C, Lussier M, Mao R, Menard P, Ooi SL, Revuelta JL, Roberts CJ, Rose M, Ross-Macdonald P, Scherens B, Schimmack G, Shafer B, Shoemaker DD, Sookhai-Mahadeo S, Storms RK, Strathern JN, Valle G, Voet M, Volckaert G, Wang CY, Ward TR, Wilhelmy J, Winzeler EA, Yang Y, Yen G, Youngman E, Yu K, Bussey H, Boeke JD, Snyder M, Philippsen P, Davis RW, Johnston M. (2002) Functional profiling of the Saccharomyces cerevisiae genome; Nature 25;418(6896):387-91.
Sambrook J., Russell D. (2001) Rapid isolation of yeast DNA. In: Sambrook J & Russell DW, eds. Molecular Cloning, a Laboratory Manual, New York: Cold Spring Harbor Laboratory Press, pp. 631-632.
Taxis C, Knop M. (2006) System of centromeric, episomal, and integrative vectors based on drug resistance markers for Saccharomyces cerevisiae; Biotechniques. 40:73-78.
Urano , et al, US Patent 8232089, Cytosolic isobutanol pathway localization for the production of isobutanol.
Chen et al (2011) Increased isobutanol production in Saccharomyces cerevisiae by overexpression of genes in valine metabolism; Biotechnology for Biofuels, 4:21.
Brat et al (2012) Cytosolic re-localization and optimization of valine synthesis and catabolism enables increased isobutanol production with the yeast Saccharomyces cerevisiae; Biotechnology for Biofuels, 5:65.

## Claims

1. A method for enhancing glycerol production in yeast comprising:
growing a yeast strain anaerobically in a culture medium, under conditions that cause the yeast strain to make glycerol,
wherein the yeast strain comprises a recombinant nucleic acid molecule comprising a truncated portion of a gene encoding a cytosol located acetolactate synthase activity, wherein said gene is operably linked to a non-native promoter to express said acetolactate synthase activity in the cytosol, wherein said truncated gene does not comprise a mitochondrial targeting signal.

2. The method of claim 1, wherein said truncated gene is according to SEQ ID NO: 1.

3. The method of claim 1 or 2, wherein said promoter element is an ADH1 promoter.

4. The method of any one of claims 1 to 3, wherein said recombinant nucleic acid molecule is according to SEQ ID NO: 1.

5. The method of any one of claims 1 to 4, wherein an amount of glycerol produced by said yeast strain is more than four times greater than an amount of glycerol produced by a corresponding yeast strain that does not contain said recombinant nucleic acid molecule but is otherwise identical to said yeast strain.

6. The method of any one of claims 1 to 5, wherein said yeast strain is S. cerevisiae.

## Patentansprüche

1. Verfahren zur Erhöhung der Glycerinproduktion in Hefe, umfassend:
anaerobes Kultivieren eines Hefestamms in einem Kulturmedium unter Bedingungen, die bewirken, dass der Hefestamm Glycerin produziert;
wobei der Hefestamm ein rekombinantes Nucleinsäuremolekül umfasst, das einen verkürzten Teil eines Gens umfasst, welches eine im Cytosol lokalisierte Acetolactat-Synthase-Aktivität codiert, wobei das Gen funktionell mit einem nichtnativen Promotor verknüpft ist, um die Acetolactat-Synthase-Aktivität in dem Cytosol zu exprimieren, wobei das verkürzte Gen kein mitochondriales Transportsignal umfasst.

2. Verfahren gemäß Anspruch 1, wobei das verkürzte Gen der SEQ ID Nr. 1 entspricht.

3. Verfahren gemäß Anspruch 1 oder 2, wobei das Promotorelement ein ADH1-Promotor ist.

4. Verfahren gemäß einem der Ansprüche 1 bis 3, wobei das rekombinante Nucleinsäuremolekül der SEQ ID Nr. 1 entspricht.

5. Verfahren gemäß einem der Ansprüche 1 bis 4, wobei die Menge an Glycerin, die von dem Hefestamm produziert wird, mehr als viermal so groß ist wie die Menge an Glycerin, die von einem entsprechenden Hefestamm produziert wird, der das rekombinante Nucleinsäuremolekül nicht enthält, aber ansonsten mit dem Hefestamm identisch ist.

6. Verfahren gemäß einem der Ansprüche 1 bis 5, wobei es sich bei dem Hefestamm um *S. cerevisiae* handelt.

## Revendications

1. Procédé pour améliorer la production de glycérine dans une levure comprenant :
la croissance d'une souche de levure dans des conditions anaérobies dans un milieu de culture, dans des conditions qui entraînent la fabrication de glycérine par la souche de levure,
dans lequel la souche de levure comprend une molécule d'acide nucléique recombinante comprenant une partie tronquée d'un gène codant pour une activité acétolactate synthase localisée dans le cytosol, où ledit gène est en liaison fonctionnelle avec un promoteur non natif pour exprimer ladite activité acétolactate synthase dans le cytosol, où ledit gène tronqué ne comprend pas de signal de ciblage mitochondrial.

2. Procédé selon la revendication 1, dans lequel ledit gène tronqué est selon SEQ ID NO:1.

3. Procédé selon la revendication 1 ou 2, dans lequel ledit élément promoteur est un promoteur ADH1.

4. Procédé selon l'une quelconque des revendications 1 à 3, dans lequel ladite molécule d'acide nucléique recombinante est selon SEQ ID NO: 1.

5. Procédé selon l'une quelconque des revendications 1 à 4, dans lequel une quantité de glycérine produite par ladite souche de levure est plus de quatre fois plus importante qu'une quantité de glycérine produite par une souche de levure correspondante qui ne contient pas ladite molécule d'acide nucléique recombinante mais est autrement identique à ladite souche de levure.

6. Procédé selon l'une quelconque des revendications 1 à 5, dans lequel ladite souche de levure est S. cerevisiae.


### SEQUENCE LISTING

<110> Archer Daniels Midland Company
<120> Recombinant Strains of Saccharomyces cerevisiae Overproducing
   Glycerol
<130> File Reference
<150> US 61/915,252
   <151> 2013-12-12
<160> 37
<170> PatentIn version 3.5
<210> 1
   <211> 2082
   <212> DNA
   <213> Saccharomyces cerevisiae
<400> 1
   atggagcctg ctccaagttt caatgttgat ccattagaac agcccgctga accttcaaaa      60
   ttggctaaga aactacgcgc tgagcctgac atggatacct ctttcgtcgg tttaactggt     120
   ggtcaaatat ttaacgaaat gatgtccaga caaaacgttg atactgtatt tggttatcca     180
   ggtggtgcta tcctacctgt ttacgatgcc attcataaca gtgataaatt caacttcgtt     240
   cttccaaaac acgaacaagg tgccggtcac atggcagaag gctacgccag agcttctggt     300
   aaaccaggtg ttgtcttggt tacttctggg ccaggtgcca ccaatgtcgt tactccaatg     360
   gcagatgcct ttgcagacgg gattccaatg gttgtcttta cagggcaagt cccaactagt     420
   gctatcggta ctgatgcttt ccaagaggct gacgtcgttg gtatttctag atcttgtacg     480
   aaatggaatg tcatggtcaa gtccgtggaa gaattgccat tgcgtattaa cgaggctttt     540
   gaaattgcca cgagcggtag accgggacca gtcttggtcg atttaccaaa ggatgttaca     600
   gcagctatct taagaaatcc aattccaaca aaaacaactc ttccatcaaa cgcactaaac     660
   caattaacca gtcgcgcaca agatgaattt gtcatgcaaa gtatcaataa agcagcagat     720
   ttgatcaact tggcaaagaa acctgtctta tacgtcggtg ctggtatttt aaaccatgca     780
   gatggtccaa gattactaaa agaattaagt gaccgtgctc aaatacctgt caccactact     840
   ttacaaggtt taggttcatt cgaccaagaa gatccaaaat cattggatat gcttggtatg     900
   cacggttgtg ctactgccaa cctggcagtg caaaatgccg acttgataat tgcagttggt     960
   gctagattcg acgaccgtgt cactggtaat atttctaaat tcgctccaga agctcgtcgt    1020
   gcagctgccg agggtagagg tggtattatt catttcgagg ttagtccaaa aaacataaac    1080
   aaggttgttc aaactcaaat agcagtggaa ggtgatgcta cgaccaatct gggcaaaatg    1140
   atgtcaaaga ttttcccagt taaggagagg tctgaatggt ttgctcaaat aaataaatgg    1200
   aagaaggaat acccatacgc ttatatggag gagactccag gatctaaaat taaaccacag    1260
   acggttataa agaaactatc caaggttgcc aacgacacag gaagacatgt cattgttaca 1320
   acgggtgtgg ggcaacatca aatgtgggct gctcaacact ggacatggag aaatccacat 1380
   actttcatca catcaggtgg tttaggtacg atgggttacg gtctccctgc cgccatcggt 1440
   gctcaagttg caaagccaga atctttggtt attgacattg atggtgacgc atcctttaac 1500
   atgactctaa cggaattgag ttctgccgtt caagctggta ctccagtgaa gattttgatt 1560
   ttgaacaatg aagagcaagg tatggttact caatggcaat ccctgttcta cgaacatcgt 1620
   tattcccaca cacatcaatt gaaccctgat ttcataaaac tagcggaggc tatgggttta 1680
   aaaggtttaa gagtcaagaa gcaagaggaa ttggacgcta agttgaaaga attcgtttct 1740
   accaagggcc cagttttgct tgaagtggaa gttgataaaa aagttcctgt tttgccaatg 1800
   gtggcaggtg gtagcggtct agacgagttc ataaattttg acccagaagt tgaaagacaa 1860
   cagactgaat tacgtcataa gcgtacaggc ggtaagcact gaatttcaaa aacatttatt 1920
   tcaaaagcat tttcagtaaa aaatgcagac tttattatta tttaatcgtg cttcttatat 1980
   atgacattct accaaatcgg tagtcatgta tatttttttc gtatatactt tatatatttt 2040
   tttctaaaaa actaatgacg gctaaaatta agtcatagat ga 2082
<210> 2
   <211> 633
   <212> PRT
   <213> Saccharomyces cerevisiae
<400> 2
   Met Glu Pro Ala Pro Ser Phe Asn Val Asp Pro Leu Glu Gln Pro Ala
   1               5                   10                  15
   Glu Pro Ser Lys Leu Ala Lys Lys Leu Arg Ala Glu Pro Asp Met Asp
               20                  25                  30
   Thr Ser Phe Val Gly Leu Thr Gly Gly Gln Ile Phe Asn Glu Met Met
           35                  40                  45
   Ser Arg Gln Asn Val Asp Thr Val Phe Gly Tyr Pro Gly Gly Ala Ile
       50                  55                  60
   Leu Pro Val Tyr Asp Ala Ile His Asn Ser Asp Lys Phe Asn Phe Val
   65                  70                  75                  80
   Leu Pro Lys His Glu Gln Gly Ala Gly His Met Ala Glu Gly Tyr Ala
                   85                  90                  95
   Arg Ala Ser Gly Lys Pro Gly Val Val Leu Val Thr Ser Gly Pro Gly
               100                 105                 110
   Ala Thr Asn Val Val Thr Pro Met Ala Asp Ala Phe Ala Asp Gly Ile
           115                 120                 125
   Pro Met Val Val Phe Thr Gly Gln Val Pro Thr Ser Ala Ile Gly Thr
       130                 135                 140
   Asp Ala Phe Gln Glu Ala Asp Val Val Gly Ile Ser Arg Ser Cys Thr
   145                 150                 155                 160
   Lys Trp Asn Val Met Val Lys Ser Val Glu Glu Leu Pro Leu Arg Ile
                   165                 170                 175
   Asn Glu Ala Phe Glu Ile Ala Thr Ser Gly Arg Pro Gly Pro Val Leu
               180                 185                 190
   Val Asp Leu Pro Lys Asp Val Thr Ala Ala Ile Leu Arg Asn Pro Ile
           195                 200                 205
   Pro Thr Lys Thr Thr Leu Pro Ser Asn Ala Leu Asn Gln Leu Thr Ser
       210                 215                 220
   Arg Ala Gln Asp Glu Phe Val Met Gln Ser Ile Asn Lys Ala Ala Asp
   225                 230                 235                 240
   Leu Ile Asn Leu Ala Lys Lys Pro Val Leu Tyr Val Gly Ala Gly Ile
                   245                 250                 255
   Leu Asn His Ala Asp Gly Pro Arg Leu Leu Lys Glu Leu Ser Asp Arg
               260                 265                 270
   Ala Gln Ile Pro Val Thr Thr Thr Leu Gln Gly Leu Gly Ser Phe Asp
           275                 280                 285
   Gln Glu Asp Pro Lys Ser Leu Asp Met Leu Gly Met His Gly Cys Ala
       290                 295                 300
   Thr Ala Asn Leu Ala Val Gln Asn Ala Asp Leu Ile Ile Ala Val Gly
   305                 310                 315                 320
   Ala Arg Phe Asp Asp Arg Val Thr Gly Asn Ile Ser Lys Phe Ala Pro
                   325                 330                 335
   Glu Ala Arg Arg Ala Ala Ala Glu Gly Arg Gly Gly Ile Ile His Phe
               340                 345                 350
   Glu Val Ser Pro Lys Asn Ile Asn Lys Val Val Gln Thr Gln Ile Ala
   355 360 365
   Val Glu Gly Asp Ala Thr Thr Asn Leu Gly Lys Met Met Ser Lys Ile
   370 375 380
   Phe Pro Val Lys Glu Arg Ser Glu Trp Phe Ala Gln Ile Asn Lys Trp
   385 390 395 400
   Lys Lys Glu Tyr Pro Tyr Ala Tyr Met Glu Glu Thr Pro Gly Ser Lys
   405 410 415
   Ile Lys Pro Gln Thr Val Ile Lys Lys Leu Ser Lys Val Ala Asn Asp
   420 425 430
   Thr Gly Arg His Val Ile Val Thr Thr Gly Val Gly Gln His Gln Met
   435 440 445
   Trp Ala Ala Gln His Trp Thr Trp Arg Asn Pro His Thr Phe Ile Thr
   450 455 460
   Ser Gly Gly Leu Gly Thr Met Gly Tyr Gly Leu Pro Ala Ala Ile Gly
   465 470 475 480
   Ala Gln Val Ala Lys Pro Glu Ser Leu Val Ile Asp Ile Asp Gly Asp
   485 490 495
   Ala Ser Phe Asn Met Thr Leu Thr Glu Leu Ser Ser Ala Val Gln Ala
   500 505 510
   Gly Thr Pro Val Lys Ile Leu Ile Leu Asn Asn Glu Glu Gln Gly Met
   515 520 525
   Val Thr Gln Trp Gln Ser Leu Phe Tyr Glu His Arg Tyr Ser His Thr
   530 535 540
   His Gln Leu Asn Pro Asp Phe Ile Lys Leu Ala Glu Ala Met Gly Leu
   545 550 555 560
   Lys Gly Leu Arg Val Lys Lys Gln Glu Glu Leu Asp Ala Lys Leu Lys
   565 570 575
   Glu Phe Val Ser Thr Lys Gly Pro Val Leu Leu Glu Val Glu Val Asp
   580 585 590
   Lys Lys Val Pro Val Leu Pro Met Val Ala Gly Gly Ser Gly Leu Asp
   595 600 605
   Glu Phe Ile Asn Phe Asp Pro Glu Val Glu Arg Gln Gln Thr Glu Leu
   610 615 620
   Arg His Lys Arg Thr Gly Gly Lys His
   625 630
<210> 3
   <211> 2633
   <212> DNA
   <213> Saccharomyces cerevisiae
<400> 3
   atgatcagac aatctacgct aaaaaacttc gctattaagc gttgctttca acatatagca      60
   taccgcaaca cacctgccat gagatcagta gctctcgcgc agcgctttta tagttcgtct     120
   tcccgttatt acagtgcgtc tccattacca gcctctaaaa ggccagagcc tgctccaagt     180
   ttcaatgttg atccattaga acagcccgct gaaccttcaa aattggctaa gaaactacgc     240
   gctgagcctg acatggatac ctctttcgtc ggtttaactg gtggtcaaat atttaacgaa     300
   atgatgtcca gacaaaacgt tgatactgta tttggttatc caggtggtgc tatcctacct     360
   gtttacgatg ccattcataa cagtgataaa ttcaacttcg ttcttccaaa acacgaacaa     420
   ggtgccggtc acatggcaga aggctacgcc agagcttctg gtaaaccagg tgttgtcttg     480
   gttacttctg ggccaggtgc caccaatgtc gttactccaa tggcagatgc ctttgcagac     540
   gggattccaa tggttgtctt tacagggcaa gtcccaacta gtgctatcgg tactgatgct     600
   ttccaagagg ctgacgtcgt tggtatttct agatcttgta cgaaatggaa tgtcatggtc     660
   aagtccgtgg aagaattgcc attgcgtatt aacgaggctt ttgaaattgc cacgagcggt     720
   agaccgggac cagtcttggt cgatttacca aaggatgtta cagcagctat cttaagaaat     780
   ccaattccaa caaaaacaac tcttccatca aacgcactaa accaattaac cagtcgcgca     840
   caagatgaat ttgtcatgca aagtatcaat aaagcagcag atttgatcaa cttggcaaag     900
   aaacctgtct tatacgtcgg tgctggtatt ttaaaccatg cagatggtcc aagattacta     960
   aaagaattaa gtgaccgtgc tcaaatacct gtcaccacta ctttacaagg tttaggttca    1020
   ttcgaccaag aagatccaaa atcattggat atgcttggta tgcacggttg tgctactgcc    1080
   aacctggcag tgcaaaatgc cgacttgata attgcagttg gtgctagatt cgacgaccgt    1140
   gtcactggta atatttctaa attcgctcca gaagctcgtc gtgcagctgc cgagggtaga    1200
   ggtggtatta ttcatttcga ggttagtcca aaaaacataa acaaggttgt tcaaactcaa    1260
   atagcagtgg aaggtgatgc tacgaccaat ctgggcaaaa tgatgtcaaa gattttccca    1320
   gttaaggaga ggtctgaatg gtttgctcaa ataaataaat ggaagaagga atacccatac    1380
   gcttatatgg aggagactcc aggatctaaa attaaaccac agacggttat aaagaaacta    1440
   tccaaggttg ccaacgacac aggaagacat gtcattgtta caacgggtgt ggggcaacat    1500
   caaatgtggg ctgctcaaca ctggacatgg agaaatccac atactttcat cacatcaggt 1560
   ggtttaggta cgatgggtta cggtctccct gccgccatcg gtgctcaagt tgcaaagcca 1620
   gaatctttgg ttattgacat tgatggtgac gcatccttta acatgactct aacggaattg 1680
   agttctgccg ttcaagctgg tactccagtg aagattttga ttttgaacaa tgaagagcaa 1740
   ggtatggtta ctcaatggca atccctgttc tacgaacatc gttattccca cacacatcaa 1800
   ttgaaccctg atttcataaa actagcggag gctatgggtt taaaaggttt aagagtcaag 1860
   aagcaagagg aattggacgc taagttgaaa gaattcgttt ctaccaaggg cccagttttg 1920
   cttgaagtgg aagttgataa aaaagttcct gttttgccaa tggtggcagg tggtagcggt 1980
   ctagacgagt tcataaattt tgacccagaa gttgaaagac aacagactga attacgtcat 2040
   aagcgtacag gcggtaagca ctgaatttca aaaacattta tttcaaaagc attttcagta 2100
   aaaaatgcag actttattat tatttaatcg tgcttcttat atatgacatt ctaccaaatc 2160
   ggtagtcatg tatatttttt tcgtatatac tttatatatt tttttctaaa aaactaatga 2220
   cggctaaaat taagtcatag atgaataata agttcaattc aagtgagttg gtagtatttg 2280
   ataaatctaa agtggatacg tagcatatgt attcaaatgg tgtgttttaa ctcgtcggac 2340
   acgacttctt tctcgttttg tcaatcatca aaaatatttc tcgaaaaggg gccgtcaaaa 2400
   attgcaagct tggaaaatac aaactggact atgtatttaa aggttatagt ttataataaa 2460
   gcataagaac agtggccaat acgaatttaa ccgctttata gaaatggcta tcttaaaaag 2520
   aggagctaga aaaaaggtac atcaggagcc agctaaacgc tctgcgaata tcaagaaagc 2580
   tacttttgat tcctcgaaga agaaagaagt tggtgtgtct gatctgacgc ttc 2633
<210> 4
   <211> 661
   <212> PRT
   <213> Saccharomyces cerevisiae
<400> 4
   Met Arg Ser Val Ala Leu Ala Gln Arg Phe Tyr Ser Ser Ser Ser Arg
   1 5 10 15
   Tyr Tyr Ser Ala Ser Pro Leu Pro Ala Ser Lys Arg Pro Glu Pro Ala
   20 25 30
   Pro Ser Phe Asn Val Asp Pro Leu Glu Gln Pro Ala Glu Pro Ser Lys
   35 40 45
   Leu Ala Lys Lys Leu Arg Ala Glu Pro Asp Met Asp Thr Ser Phe Val
   50 55 60
   Gly Leu Thr Gly Gly Gln Ile Phe Asn Glu Met Met Ser Arg Gln Asn
   65                  70                  75                  80
   Val Asp Thr Val Phe Gly Tyr Pro Gly Gly Ala Ile Leu Pro Val Tyr
                   85                  90                  95
   Asp Ala Ile His Asn Ser Asp Lys Phe Asn Phe Val Leu Pro Lys His
               100                 105                 110
   Glu Gln Gly Ala Gly His Met Ala Glu Gly Tyr Ala Arg Ala Ser Gly
           115                 120                 125
   Lys Pro Gly Val Val Leu Val Thr Ser Gly Pro Gly Ala Thr Asn Val
       130                 135                 140
   Val Thr Pro Met Ala Asp Ala Phe Ala Asp Gly Ile Pro Met Val Val
   145                 150                 155                 160
   Phe Thr Gly Gln Val Pro Thr Ser Ala Ile Gly Thr Asp Ala Phe Gln
                   165                 170                 175
   Glu Ala Asp Val Val Gly Ile Ser Arg Ser Cys Thr Lys Trp Asn Val
               180                 185                 190
   Met Val Lys Ser Val Glu Glu Leu Pro Leu Arg Ile Asn Glu Ala Phe
           195                 200                 205
   Glu Ile Ala Thr Ser Gly Arg Pro Gly Pro Val Leu Val Asp Leu Pro
       210                 215                 220
   Lys Asp Val Thr Ala Ala Ile Leu Arg Asn Pro Ile Pro Thr Lys Thr
   225                 230                 235                 240
   Thr Leu Pro Ser Asn Ala Leu Asn Gln Leu Thr Ser Arg Ala Gln Asp
                   245                 250                 255
   Glu Phe Val Met Gln Ser Ile Asn Lys Ala Ala Asp Leu Ile Asn Leu
               260                 265                 270
   Ala Lys Lys Pro Val Leu Tyr Val Gly Ala Gly Ile Leu Asn His Ala
           275                 280                 285
   Asp Gly Pro Arg Leu Leu Lys Glu Leu Ser Asp Arg Ala Gln Ile Pro
       290                 295                 300
   Val Thr Thr Thr Leu Gln Gly Leu Gly Ser Phe Asp Gln Glu Asp Pro
   305                 310                 315                 320
   Lys Ser Leu Asp Met Leu Gly Met His Gly Cys Ala Thr Ala Asn Leu
                   325                 330                 335
   Ala Val Gln Asn Ala Asp Leu Ile Ile Ala Val Gly Ala Arg Phe Asp
               340                 345                 350
   Asp Arg Val Thr Gly Asn Ile Ser Lys Phe Ala Pro Glu Ala Arg Arg
           355                 360                 365
   Ala Ala Ala Glu Gly Arg Gly Gly Ile Ile His Phe Glu Val Ser Pro
       370                 375                 380
   Lys Asn Ile Asn Lys Val Val Gln Thr Gln Ile Ala Val Glu Gly Asp
   385                 390                 395                 400
   Ala Thr Thr Asn Leu Gly Lys Met Met Ser Lys Ile Phe Pro Val Lys
                   405                 410                 415
   Glu Arg Ser Glu Trp Phe Ala Gln Ile Asn Lys Trp Lys Lys Glu Tyr
               420                 425                 430
   Pro Tyr Ala Tyr Met Glu Glu Thr Pro Gly Ser Lys Ile Lys Pro Gln
           435                 440                 445
   Thr Val Ile Lys Lys Leu Ser Lys Val Ala Asn Asp Thr Gly Arg His
       450                 455                 460
   Val Ile Val Thr Thr Gly Val Gly Gln His Gln Met Trp Ala Ala Gln
   465                 470                 475                 480
   His Trp Thr Trp Arg Asn Pro His Thr Phe Ile Thr Ser Gly Gly Leu
                   485                 490                 495
   Gly Thr Met Gly Tyr Gly Leu Pro Ala Ala Ile Gly Ala Gln Val Ala
               500                 505                 510
   Lys Pro Glu Ser Leu Val Ile Asp Ile Asp Gly Asp Ala Ser Phe Asn
           515                 520                 525
   Met Thr Leu Thr Glu Leu Ser Ser Ala Val Gln Ala Gly Thr Pro Val
       530                 535                 540
   Lys Ile Leu Ile Leu Asn Asn Glu Glu Gln Gly Met Val Thr Gln Trp
   545                 550                 555                 560
   Gln Ser Leu Phe Tyr Glu His Arg Tyr Ser His Thr His Gln Leu Asn
                   565                 570                 575
   Pro Asp Phe Ile Lys Leu Ala Glu Ala Met Gly Leu Lys Gly Leu Arg
   580 585 590
   Val Lys Lys Gln Glu Glu Leu Asp Ala Lys Leu Lys Glu Phe Val Ser
   595 600 605
   Thr Lys Gly Pro Val Leu Leu Glu Val Glu Val Asp Lys Lys Val Pro
   610 615 620
   Val Leu Pro Met Val Ala Gly Gly Ser Gly Leu Asp Glu Phe Ile Asn
   625 630 635 640
   Phe Asp Pro Glu Val Glu Arg Gln Gln Thr Glu Leu Arg His Lys Arg
   645 650 655
   Thr Gly Gly Lys His
   660
<210> 5
   <211> 691
   <212> DNA
   <213> Saccharomyces cerevisiae
<400> 5
   atatggactt cctcttttct ggcaaccaaa cccatacatc gggattccta taataccttc 60
   gttggtctcc ctaacatgta ggtggcggag gggagatata caatagaaca gataccagac 120
   aagacataat gggctaaaca agactacacc aattacactg cctcattgat ggtggtacat 180
   aacgaactaa tactgtagcc ctagacttga tagccatcat catatcgaag tttcactacc 240
   ctttttccat ttgccatcta ttgaagtaat aataggcgca tgcaacttct tttctttttt 300
   tttcttttct ctctcccccg ttgttgtctc accatatccg caatgacaaa aaaatgatgg 360
   aagacactaa aggaaaaaat taacgacaaa gacagcacca acagatgtcg ttgttccaga 420
   gctgatgagg ggtatctcga agcacacgaa actttttcct tccttcattc acgcacacta 480
   ctctctaatg agcaacggta tacggccttc cttccagtta cttgaatttg aaataaaaaa 540
   aagtttgctg tcttgctatc aagtataaat agacctgcaa ttattaatct tttgtttcct 600
   cgtcattgtt ctcgttccct ttcttccttg tttctttttc tgcacaatat ttcaagctat 660
   accaagcata caatcaacta tctcatatac a 691
<210> 6
   <211> 605
   <212> DNA
   <213> Saccharomyces cerevisiae
<400> 6
   tgagggagac ctaactacat agtgtttaaa gattacggat atttaactta cttagaataa        60
   tgccattttt ttgagttata ataatcctac gttagtgtga gcgggattta aactgtgagg       120
   accttaatac attcagacac ttctgcggta tcaccctact tattcccttc gagattatat       180
   ctaggaaccc atcaggttgg tggaagatta cccgttctaa gacttttcag cttcctctat       240
   tgatgttaca cctggacacc ccttttctgg catccagttt ttaatcttca gtggcatgtg       300
   agattctccg aaattaatta aagcaatcac acaattctct cggataccac ctcggttgaa       360
   actgacaggt ggtttgttac gcatgctaat gcaaaggagc ctatatacct ttggctcggc       420
   tgctgtaaca gggaatataa agggcagcat aatttaggag tttagtgaac ttgcaacatt       480
   tactattttc ccttcttacg taaatatttt tctttttaat tctaaatcaa tctttttcaa       540
   ttttttgttt gtattctttt cttgcttaaa tctataacta caaaaaacac atacataaac       600
   taaaa                                                                   605
<210> 7
   <211> 1391
   <212> DNA
   <213> Streptomyces noursei
<400> 7
   gtgatgacgg tgaaaacctc tgacacatgc agctcccgga gacggtcaca gcttgtctgt 60
   aagcggatgc cgggagcaga caagcccgtc agggcgcgtc agcgggtgtt ggcgggtgtc 120
   ggggctggct taactatgcg gcatcagagc agattgtact gagagtgcag cgacatggag 180
   gcccagaata ccctccttga cagtcttgac gtgcgcagct caggggcatg atgtgactgt 240
   cgcccgtaca tttagcccat acatccccat gtataatcat ttgcatccat acattttgat 300
   ggccgcacgg cgcgaagcaa aaattacggc tcctcgctgc agacctgcga gcagggaaac 360
   gctcccctca cagacgcgtt gaattgtccc cacgccgcgc ccctgtagag aaatataaaa 420
   ggttaggatt tgccactgag gttcttcttt catatacttc cttttaaaat cttgctagga 480
   tacagttctc acatcacatc cgaacataaa caaccatggg taccactctt gacgacacgg 540
   cttaccggta ccgcaccagt gtcccggggg acgccgaggc catcgaggca ctggatgggt 600
   ccttcaccac cgacaccgtc ttccgcgtca ccgccaccgg ggacggcttc accctgcggg 660
   aggtgccggt ggacccgccc ctgaccaagg tgttccccga cgacgaatcg gacgacgaat 720
   cggacgacgg ggaggacggc gacccggact cccggacgtt cgtcgcgtac ggggacgacg 780
   gcgacctggc gggcttcgtg gtcatctcgt actcggcgtg gaaccgccgg ctgaccgtcg 840
   aggacatcga ggtcgccccg gagcaccggg ggcacggggt cgggcgcgcg ttgatggggc 900
   tcgcgacgga gttcgccggc gagcggggcg ccgggcacct ctggctggag gtcaccaacg 960
   tcaacgcacc ggcgatccac gcgtaccggc ggatggggtt caccctctgc ggcctggaca 1020
   ccgccctgta cgacggcacc gcctcggacg gcgagcggca ggcgctctac atgagcatgc 1080
   cctgccccta atctcgaggc gaatttctta tgatttatga tttttattat taaataagtt 1140
   ataaaaaaaa taagtgtata caaattttaa agtgactctt aggttttaaa acgaaaattc 1200
   ttgttcttga gtaactcttt cctgtaggtc aggttgcttt ctcaggtata gcatgaggtc 1260
   gctcttattg accacacctc taccggcatg ccgagcaaat gcctgcaaat cgctccccat 1320
   ttcacccaat tgtagatatg ctaactccag caatgagttg atgaatctcg gtgtgtattt 1380
   tatgtcctca g 1391
<210> 8
   <211> 34
   <212> DNA
   <213> Artificial Sequence
<220> unsure
   <223> LoxP- genetic tool for marker rescue
<400> 8
   ataacttcgt atagcataca ttatacgaag ttat 34
<210> 9
   <211> 6822
   <212> DNA
   <213> Artificial Sequence
<220> unsure
   <223> pUC57-ADH1-ILV2(truncated)-natNT2 plasmid sequence
<400> 9
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt acctcgcgaa 420
   tgcatctaga tatcggatcc atatggactt cctcttttct ggcaaccaaa cccatacatc 480
   gggattccta taataccttc gttggtctcc ctaacatgta ggtggcggag gggagatata 540
   caatagaaca gataccagac aagacataat gggctaaaca agactacacc aattacactg 600
   cctcattgat ggtggtacat aacgaactaa tactgtagcc ctagacttga tagccatcat 660
   catatcgaag tttcactacc ctttttccat ttgccatcta ttgaagtaat aataggcgca 720
   tgcaacttct ttcttttttt tttcttttct ctctcccccg ttgttgtctc accatatccg 780
   caatgacaaa aaaatgatgg aagacactaa aggaaaaaat taacgacaaa gacagcacca 840
   acagatgtcg ttgttccaga gctgatgagg ggtatctcga agcacacgaa actttttcct 900
   tccttcattc acgcacacta ctctctaatg agcaacggta tacggccttc cttccagtta 960
   cttgaatttg aaataaaaaa aagtttgctg tcttgctatc aagtataaat agacctgcaa 1020
   ttattaatct tttgtttcct cgtcattgtt ctcgttccct ttcttccttg tttctttttc 1080
   tgcacaatat ttcaagctat accaagcata caatcaacta tctcatatac agtcgacatg 1140
   gagcctgctc caagtttcaa tgttgatcca ttagaacagc ccgctgaacc ttcaaaattg 1200
   gctaagaaac tacgcgctga gcctgacatg gatacctctt tcgtcggttt aactggtggt 1260
   caaatattta acgaaatgat gtccagacaa aacgttgata ctgtatttgg ttatccaggt 1320
   ggtgctatcc tacctgttta cgatgccatt cataacagtg ataaattcaa cttcgttctt 1380
   ccaaaacacg aacaaggtgc cggtcacatg gcagaaggct acgccagagc ttctggtaaa 1440
   ccaggtgttg tcttggttac ttctgggcca ggtgccacca atgtcgttac tccaatggca 1500
   gatgcctttg cagacgggat tccaatggtt gtctttacag ggcaagtccc aactagtgct 1560
   atcggtactg atgctttcca agaggctgac gtcgttggta tttctagatc ttgtacgaaa 1620
   tggaatgtca tggtcaagtc cgtggaagaa ttgccattgc gtattaacga ggcttttgaa 1680
   attgccacga gcggtagacc gggaccagtc ttggtcgatt taccaaagga tgttacagca 1740
   gctatcttaa gaaatccaat tccaacaaaa acaactcttc catcaaacgc actaaaccaa 1800
   ttaaccagtc gcgcacaaga tgaatttgtc atgcaaagta tcaataaagc agcagatttg 1860
   atcaacttgg caaagaaacc tgtcttatac gtcggtgctg gtattttaaa ccatgcagat 1920
   ggtccaagat tactaaaaga attaagtgac cgtgctcaaa tacctgtcac cactacttta 1980
   caaggtttag gttcattcga ccaagaagat ccaaaatcat tggatatgct tggtatgcac 2040
   ggttgtgcta ctgccaacct ggcagtgcaa aatgccgact tgataattgc agttggtgct 2100
   agattcgacg accgtgtcac tggtaatatt tctaaattcg ctccagaagc tcgtcgtgca 2160
   gctgccgagg gtagaggtgg tattattcat ttcgaggtta gtccaaaaaa cataaacaag 2220
   gttgttcaaa ctcaaatagc agtggaaggt gatgctacga ccaatctggg caaaatgatg 2280
   tcaaagattt tcccagttaa ggagaggtct gaatggtttg ctcaaataaa taaatggaag 2340
   aaggaatacc catacgctta tatggaggag actccaggat ctaaaattaa accacagacg 2400
   gttataaaga aactatccaa ggttgccaac gacacaggaa gacatgtcat tgttacaacg 2460
   ggtgtggggc aacatcaaat gtgggctgct caacactgga catggagaaa tccacatact 2520
   ttcatcacat caggtggttt aggtacgatg ggttacggtc tccctgccgc catcggtgct 2580
   caagttgcaa agccagaatc tttggttatt gacattgatg gtgacgcatc ctttaacatg 2640
   actctaacgg aattgagttc tgccgttcaa gctggtactc cagtgaagat tttgattttg 2700
   aacaatgaag agcaaggtat ggttactcaa tggcaatccc tgttctacga acatcgttat 2760
   tcccacacac atcaattgaa ccctgatttc ataaaactag cggaggctat gggtttaaaa 2820
   ggtttaagag tcaagaagca agaggaattg gacgctaagt tgaaagaatt cgtttctacc 2880
   aagggcccag ttttgcttga agtggaagtt gataaaaaag ttcctgtttt gccaatggtg 2940
   gcaggtggta gcggtctaga cgagttcata aattttgacc cagaagttga aagacaacag 3000
   actgaattac gtcataagcg tacaggcggt aagcactgaa tttcaaaaac atttatttca 3060
   aaagcatttt cagtaaaaaa tgcagacttt attattattt aatcgtgctt cttatatatg 3120
   acattctacc aaatcggtag tcatgtatat ttttttcgta tatactttat atattttttt 3180
   ctaaaaaact aatgacggct aaaattaagt catagatgac tgcagaggcc tgcatgcaag 3240
   cttggcgcgc cagatctata acttcgtata gcatacatta tacgaagtta tcttaactat 3300
   gcggcatcag agcagattgt actgagagtg cagcgacatg gaggcccaga ataccctcct 3360
   tgacagtctt gacgtgcgca gctcaggggc atgatgtgac tgtcgcccgt acatttagcc 3420
   catacatccc catgtataat catttgcatc catacatttt gatggccgca cggcgcgaag 3480
   caaaaattac ggctcctcgc tgcagacctg cgagcaggga aacgctcccc tcacagacgc 3540
   gttgaattgt ccccacgccg cgcccctgta gagaaatata aaaggttagg atttgccact 3600
   gaggttcttc tttcatatac ttccttttaa aatcttgcta ggatacagtt ctcacatcac 3660
   atccgaacat aaacaaccat gggtaccact cttgacgaca cggcttaccg gtaccgcacc 3720
   agtgtcccgg gggacgccga ggccatcgag gcactggatg ggtccttcac caccgacacc 3780
   gtcttccgcg tcaccgccac cggggacggc ttcaccctgc gggaggtgcc ggtggacccg 3840
   cccctgacca aggtgttccc cgacgacgaa tcggacgacg aatcggacga cggggaggac 3900
   ggcgacccgg actcccggac gttcgtcgcg tacggggacg acggcgacct ggcgggcttc 3960
   gtggtcatct cgtactcggc gtggaaccgc cggctgaccg tcgaggacat cgaggtcgcc 4020
   ccggagcacc gggggcacgg ggtcgggcgc gcgttgatgg ggctcgcgac ggagttcgcc 4080
   ggcgagcggg gcgccgggca cctctggctg gaggtcacca acgtcaacgc accggcgatc 4140
   cacgcgtacc ggcggatggg gttcaccctc tgcggcctgg acaccgccct gtacgacggc 4200
   accgcctcgg acggcgagcg gcaggcgctc tacatgagca tgccctgccc ctaatctcga 4260
   ggcgaatttc ttatgattta tgatttttat tattaaataa gttataaaaa aaataagtgt 4320
   atacaaattt taaagtgact cttaggtttt aaaacgaaaa ttcttgttct tgagtaactc 4380
   tttcctgtag gtcaggttgc tttctcaggt atagcatgag gtcgctctta ttgaccacac 4440
   ctctaccggc atgccgagca aatgcctgca aatcgctccc catttcaccc aattgtagat 4500
   atgctaactc cagcaatgag ttgatgaatc tcggataact tcgtatagca tacattatac 4560
   gaagttatag atctggcgcg ccaagcttgg cgtaatcatg gtcatagctg tttcctgtgt 4620
   gaaattgtta tccgctcaca attccacaca acatacgagc cggaagcata aagtgtaaag 4680
   cctggggtgc ctaatgagtg agctaactca cattaattgc gttgcgctca ctgcccgctt 4740
   tccagtcggg aaacctgtcg tgccagctgc attaatgaat cggccaacgc gcggggagag 4800
   gcggtttgcg tattgggcgc tcttccgctt cctcgctcac tgactcgctg cgctcggtcg 4860
   ttcggctgcg gcgagcggta tcagctcact caaaggcggt aatacggtta tccacagaat 4920
   caggggataa cgcaggaaag aacatgtgag caaaaggcca gcaaaaggcc aggaaccgta 4980
   aaaaggccgc gttgctggcg tttttccata ggctccgccc ccctgacgag catcacaaaa 5040
   atcgacgctc aagtcagagg tggcgaaacc cgacaggact ataaagatac caggcgtttc 5100
   cccctggaag ctccctcgtg cgctctcctg ttccgaccct gccgcttacc ggatacctgt 5160
   ccgcctttct cccttcggga agcgtggcgc tttctcatag ctcacgctgt aggtatctca 5220
   gttcggtgta ggtcgttcgc tccaagctgg gctgtgtgca cgaacccccc gttcagcccg 5280
   accgctgcgc cttatccggt aactatcgtc ttgagtccaa cccggtaaga cacgacttat 5340
   cgccactggc agcagccact ggtaacagga ttagcagagc gaggtatgta ggcggtgcta 5400
   cagagttctt gaagtggtgg cctaactacg gctacactag aagaacagta tttggtatct 5460
   gcgctctgct gaagccagtt accttcggaa aaagagttgg tagctcttga tccggcaaac 5520
   aaaccaccgc tggtagcggt ggtttttttg tttgcaagca gcagattacg cgcagaaaaa 5580
   aaggatctca agaagatcct ttgatctttt ctacggggtc tgacgctcag tggaacgaaa 5640
   actcacgtta agggattttg gtcatgagat tatcaaaaag gatcttcacc tagatccttt 5700
   taaattaaaa atgaagtttt aaatcaatct aaagtatata tgagtaaact tggtctgaca 5760
   gttaccaatg cttaatcagt gaggcaccta tctcagcgat ctgtctattt cgttcatcca 5820
   tagttgcctg actccccgtc gtgtagataa ctacgatacg ggagggctta ccatctggcc 5880
   ccagtgctgc aatgataccg cgagacccac gctcaccggc tccagattta tcagcaataa 5940
   accagccagc cggaagggcc gagcgcagaa gtggtcctgc aactttatcc gcctccatcc 6000
   agtctattaa ttgttgccgg gaagctagag taagtagttc gccagttaat agtttgcgca 6060
   acgttgttgc cattgctaca ggcatcgtgg tgtcacgctc gtcgtttggt atggcttcat 6120
   tcagctccgg ttcccaacga tcaaggcgag ttacatgatc ccccatgttg tgcaaaaaag 6180
   cggttagctc cttcggtcct ccgatcgttg tcagaagtaa gttggccgca gtgttatcac 6240
   tcatggttat ggcagcactg cataattctc ttactgtcat gccatccgta agatgctttt 6300
   ctgtgactgg tgagtactca accaagtcat tctgagaata gtgtatgcgg cgaccgagtt 6360
   gctcttgccc ggcgtcaata cgggataata ccgcgccaca tagcagaact ttaaaagtgc 6420
   tcatcattgg aaaacgttct tcggggcgaa aactctcaag gatcttaccg ctgttgagat 6480
   ccagttcgat gtaacccact cgtgcaccca actgatcttc agcatctttt actttcacca     6540
   gcgtttctgg gtgagcaaaa acaggaaggc aaaatgccgc aaaaaaggga ataagggcga     6600
   cacggaaatg ttgaatactc atactcttcc tttttcaata ttattgaagc atttatcagg     6660
   gttattgtct catgagcgga tacatatttg aatgtattta gaaaaataaa caaatagggg     6720
   ttccgcgcac atttccccga aaagtgccac ctgacgtcta agaaaccatt attatcatga     6780
   cattaaccta taaaaatagg cgtatcacga ggccctttcg tc                        6822
<210> 10
   <211> 7356
   <212> DNA
   <213> Artificial Sequence
<220> unsure
   <223> pUC57-natNT2-TPI1-ILV2n plasmid sequence
<400> 10
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgaatt cgagctcggt acctcgcgaa     420
   tgcatctaga tatcggatcc tctagagtga tgacggtgaa aacctctgac acatgcagct     480
   cccggagacg gtcacagctt gtctgtaagc ggatgccggg agcagacaag cccgtcaggg     540
   cgcgtcagcg ggtgttggcg ggtgtcgggg ctggcttaac tatgcggcat cagagcagat     600
   tgtactgaga gtgcagcgac atggaggccc agaataccct ccttgacagt cttgacgtgc     660
   gcagctcagg ggcatgatgt gactgtcgcc cgtacattta gcccatacat ccccatgtat     720
   aatcatttgc atccatacat tttgatggcc gcacggcgcg aagcaaaaat tacggctcct     780
   cgctgcagac ctgcgagcag ggaaacgctc ccctcacaga cgcgttgaat tgtccccacg     840
   ccgcgcccct gtagagaaat ataaaaggtt aggatttgcc actgaggttc ttctttcata     900
   tacttccttt taaaatcttg ctaggataca gttctcacat cacatccgaa cataaacaac     960
   catgggtacc actcttgacg acacggctta ccggtaccgc accagtgtcc cgggggacgc    1020
   cgaggccatc gaggcactgg atgggtcctt caccaccgac accgtcttcc gcgtcaccgc    1080
   caccggggac ggcttcaccc tgcgggaggt gccggtggac ccgcccctga ccaaggtgtt    1140
   ccccgacgac gaatcggacg acgaatcgga cgacggggag gacggcgacc cggactcccg    1200
   gacgttcgtc gcgtacgggg acgacggcga cctggcgggc ttcgtggtca tctcgtactc    1260
   ggcgtggaac cgccggctga ccgtcgagga catcgaggtc gccccggagc accgggggca 1320
   cggggtcggg cgcgcgttga tggggctcgc gacggagttc gccggcgagc ggggcgccgg 1380
   gcacctctgg ctggaggtca ccaacgtcaa cgcaccggcg atccacgcgt accggcggat 1440
   ggggttcacc ctctgcggcc tggacaccgc cctgtacgac ggcaccgcct cggacggcga 1500
   gcggcaggcg ctctacatga gcatgccctg cccctaatct cgaggcgaat ttcttatgat 1560
   ttatgatttt tattattaaa taagttataa aaaaaataag tgtatacaaa ttttaaagtg 1620
   actcttaggt tttaaaacga aaattcttgt tcttgagtaa ctctttcctg taggtcaggt 1680
   tgctttctca ggtatagcat gaggtcgctc ttattgacca cacctctacc ggcatgccga 1740
   gcaaatgcct gcaaatcgct ccccatttca cccaattgta gatatgctaa ctccagcaat 1800
   gagttgatga atctcggtgt gtattttatg tcctcagtct agaggatcct gagggagacc 1860
   taactacata gtgtttaaag attacggata tttaacttac ttagaataat gccatttttt 1920
   tgagttataa taatcctacg ttagtgtgag cgggatttaa actgtgagga ccttaataca 1980
   ttcagacact tctgcggtat caccctactt attcccttcg agattatatc taggaaccca 2040
   tcaggttggt ggaagattac ccgttctaag acttttcagc ttcctctatt gatgttacac 2100
   ctggacaccc cttttctggc atccagtttt taatcttcag tggcatgtga gattctccga 2160
   aattaattaa agcaatcaca caattctctc ggataccacc tcggttgaaa ctgacaggtg 2220
   gtttgttacg catgctaatg caaaggagcc tatatacctt tggctcggct gctgtaacag 2280
   ggaatataaa gggcagcata atttaggagt ttagtgaact tgcaacattt actattttcc 2340
   cttcttacgt aaatattttt ctttttaatt ctaaatcaat ctttttcaat tttttgtttg 2400
   tattcttttc ttgcttaaat ctataactac aaaaaacaca tacataaact aaaaggtacc 2460
   atgatcagac aatctacgct aaaaaacttc gctattaagc gttgctttca acatatagca 2520
   taccgcaaca cacctgccat gagatcagta gctctcgcgc agcgctttta tagttcgtct 2580
   tcccgttatt acagtgcgtc tccattacca gcctctaaaa ggccagagcc tgctccaagt 2640
   ttcaatgttg atccattaga acagcccgct gaaccttcaa aattggctaa gaaactacgc 2700
   gctgagcctg acatggatac ctctttcgtc ggtttaactg gtggtcaaat atttaacgaa 2760
   atgatgtcca gacaaaacgt tgatactgta tttggttatc caggtggtgc tatcctacct 2820
   gtttacgatg ccattcataa cagtgataaa ttcaacttcg ttcttccaaa acacgaacaa 2880
   ggtgccggtc acatggcaga aggctacgcc agagcttctg gtaaaccagg tgttgtcttg 2940
   gttacttctg ggccaggtgc caccaatgtc gttactccaa tggcagatgc ctttgcagac 3000
   gggattccaa tggttgtctt tacagggcaa gtcccaacta gtgctatcgg tactgatgct 3060
   ttccaagagg ctgacgtcgt tggtatttct agatcttgta cgaaatggaa tgtcatggtc 3120
   aagtccgtgg aagaattgcc attgcgtatt aacgaggctt ttgaaattgc cacgagcggt 3180
   agaccgggac cagtcttggt cgatttacca aaggatgtta cagcagctat cttaagaaat 3240
   ccaattccaa caaaaacaac tcttccatca aacgcactaa accaattaac cagtcgcgca 3300
   caagatgaat ttgtcatgca aagtatcaat aaagcagcag atttgatcaa cttggcaaag 3360
   aaacctgtct tatacgtcgg tgctggtatt ttaaaccatg cagatggtcc aagattacta 3420
   aaagaattaa gtgaccgtgc tcaaatacct gtcaccacta ctttacaagg tttaggttca 3480
   ttcgaccaag aagatccaaa atcattggat atgcttggta tgcacggttg tgctactgcc 3540
   aacctggcag tgcaaaatgc cgacttgata attgcagttg gtgctagatt cgacgaccgt 3600
   gtcactggta atatttctaa attcgctcca gaagctcgtc gtgcagctgc cgagggtaga 3660
   ggtggtatta ttcatttcga ggttagtcca aaaaacataa acaaggttgt tcaaactcaa 3720
   atagcagtgg aaggtgatgc tacgaccaat ctgggcaaaa tgatgtcaaa gattttccca 3780
   gttaaggaga ggtctgaatg gtttgctcaa ataaataaat ggaagaagga atacccatac 3840
   gcttatatgg aggagactcc aggatctaaa attaaaccac agacggttat aaagaaacta 3900
   tccaaggttg ccaacgacac aggaagacat gtcattgtta caacgggtgt ggggcaacat 3960
   caaatgtggg ctgctcaaca ctggacatgg agaaatccac atactttcat cacatcaggt 4020
   ggtttaggta cgatgggtta cggtctccct gccgccatcg gtgctcaagt tgcaaagcca 4080
   gaatctttgg ttattgacat tgatggtgac gcatccttta acatgactct aacggaattg 4140
   agttctgccg ttcaagctgg tactccagtg aagattttga ttttgaacaa tgaagagcaa 4200
   ggtatggtta ctcaatggca atccctgttc tacgaacatc gttattccca cacacatcaa 4260
   ttgaaccctg atttcataaa actagcggag gctatgggtt taaaaggttt aagagtcaag 4320
   aagcaagagg aattggacgc taagttgaaa gaattcgttt ctaccaaggg cccagttttg 4380
   cttgaagtgg aagttgataa aaaagttcct gttttgccaa tggtggcagg tggtagcggt 4440
   ctagacgagt tcataaattt tgacccagaa gttgaaagac aacagactga attacgtcat 4500
   aagcgtacag gcggtaagca ctgaatttca aaaacattta tttcaaaagc attttcagta 4560
   aaaaatgcag actttattat tatttaatcg tgcttcttat atatgacatt ctaccaaatc 4620
   ggtagtcatg tatatttttt tcgtatatac tttatatatt tttttctaaa aaactaatga 4680
   cggctaaaat taagtcatag atgaataata agttcaattc aagtgagttg gtagtatttg 4740
   ataaatctaa agtggatacg tagcatatgt attcaaatgg tgtgttttaa ctcgtcggac 4800
   acgacttctt tctcgttttg tcaatcatca aaaatatttc tcgaaaaggg gccgtcaaaa 4860
   attgcaagct tggaaaatac aaactggact atgtatttaa aggttatagt ttataataaa 4920
   gcataagaac agtggccaat acgaatttaa ccgctttata gaaatggcta tcttaaaaag 4980
   aggagctaga aaaaaggtac atcaggagcc agctaaacgc tctgcgaata tcaagaaagc 5040
   tacttttgat tcctcgaaga agaaagaagt tggtgtgtct gatctgacgc ttcgtcgact 5100
   gcagaggcct gcatgcaagc ttggcgtaat catggtcata gctgtttcct gtgtgaaatt 5160
   gttatccgct cacaattcca cacaacatac gagccggaag cataaagtgt aaagcctggg 5220
   gtgcctaatg agtgagctaa ctcacattaa ttgcgttgcg ctcactgccc gctttccagt 5280
   cgggaaacct gtcgtgccag ctgcattaat gaatcggcca acgcgcgggg agaggcggtt 5340
   tgcgtattgg gcgctcttcc gcttcctcgc tcactgactc gctgcgctcg gtcgttcggc 5400
   tgcggcgagc ggtatcagct cactcaaagg cggtaatacg gttatccaca gaatcagggg 5460
   ataacgcagg aaagaacatg tgagcaaaag gccagcaaaa ggccaggaac cgtaaaaagg 5520
   ccgcgttgct ggcgtttttc cataggctcc gcccccctga cgagcatcac aaaaatcgac 5580
   gctcaagtca gaggtggcga aacccgacag gactataaag ataccaggcg tttccccctg 5640
   gaagctccct cgtgcgctct cctgttccga ccctgccgct taccggatac ctgtccgcct 5700
   ttctcccttc gggaagcgtg gcgctttctc atagctcacg ctgtaggtat ctcagttcgg 5760
   tgtaggtcgt tcgctccaag ctgggctgtg tgcacgaacc ccccgttcag cccgaccgct 5820
   gcgccttatc cggtaactat cgtcttgagt ccaacccggt aagacacgac ttatcgccac 5880
   tggcagcagc cactggtaac aggattagca gagcgaggta tgtaggcggt gctacagagt 5940
   tcttgaagtg gtggcctaac tacggctaca ctagaagaac agtatttggt atctgcgctc 6000
   tgctgaagcc agttaccttc ggaaaaagag ttggtagctc ttgatccggc aaacaaacca 6060
   ccgctggtag cggtggtttt tttgtttgca agcagcagat tacgcgcaga aaaaaaggat 6120
   ctcaagaaga tcctttgatc ttttctacgg ggtctgacgc tcagtggaac gaaaactcac 6180
   gttaagggat tttggtcatg agattatcaa aaaggatctt cacctagatc cttttaaatt 6240
   aaaaatgaag ttttaaatca atctaaagta tatatgagta aacttggtct gacagttacc 6300
   aatgcttaat cagtgaggca cctatctcag cgatctgtct atttcgttca tccatagttg 6360
   cctgactccc cgtcgtgtag ataactacga tacgggaggg cttaccatct ggccccagtg 6420
   ctgcaatgat accgcgagac ccacgctcac cggctccaga tttatcagca ataaaccagc 6480
   cagccggaag ggccgagcgc agaagtggtc ctgcaacttt atccgcctcc atccagtcta 6540
   ttaattgttg ccgggaagct agagtaagta gttcgccagt taatagtttg cgcaacgttg 6600
   ttgccattgc tacaggcatc gtggtgtcac gctcgtcgtt tggtatggct tcattcagct 6660
   ccggttccca acgatcaagg cgagttacat gatcccccat gttgtgcaaa aaagcggtta 6720
   gctccttcgg tcctccgatc gttgtcagaa gtaagttggc cgcagtgtta tcactcatgg 6780
   ttatggcagc actgcataat tctcttactg tcatgccatc cgtaagatgc ttttctgtga 6840
   ctggtgagta ctcaaccaag tcattctgag aatagtgtat gcggcgaccg agttgctctt 6900
   gcccggcgtc aatacgggat aataccgcgc cacatagcag aactttaaaa gtgctcatca 6960
   ttggaaaacg ttcttcgggg cgaaaactct caaggatctt accgctgttg agatccagtt 7020
   cgatgtaacc cactcgtgca cccaactgat cttcagcatc ttttactttc accagcgttt 7080
   ctgggtgagc aaaaacagga aggcaaaatg ccgcaaaaaa gggaataagg gcgacacgga 7140
   aatgttgaat actcatactc ttcctttttc aatattattg aagcatttat cagggttatt 7200
   gtctcatgag cggatacata tttgaatgta tttagaaaaa taaacaaata ggggttccgc 7260
   gcacatttcc ccgaaaagtg ccacctgacg tctaagaaac cattattatc atgacattaa 7320
   cctataaaaa taggcgtatc acgaggccct ttcgtc 7356
<210> 11
   <211> 50
   <212> DNA
   <213> Artificial Sequence
<220> primer_bind
   <223> Ko574 PCR primer
<400> 11
   caatcaacta tctcatatac agtcgacatg gagcctgctc caagtttcaa 50
<210> 12
   <211> 32
   <212> DNA
   <213> Artificial Sequence
<220> primer_bind
   <223> Ko575 PCR primer
<400> 12
   aaactgcagt catctatgac ttaattttag cc 32
<210> 13
   <211> 30
   <212> DNA
   <213> Artificial Sequence
<220> primer_bind
   <223> Ko572 PCR primer
<400> 13
   cgcggatcca tatggacttc ctcttttctg 30
<210> 14
   <211> 50
   <212> DNA
   <213> Artificial Sequence
<220> primer_bind
   <223> Ko573 PCR Primer
<400> 14
   ttgaaacttg gagcaggctc catgtcgact gtatatgaga tagttgattg 50
<210> 15
   <211> 78
   <212> DNA
   <213> Artificial Sequence
<220> primer_bind
   <223> OK19 PCR primer
<400> 15
   cccaagcttg gcgcgccaga tctataactt cgtatagcat acattatacg aagttatctt 60
   aactatgcgg catcagag 78
<210> 16
   <211> 76
   <212> DNA
   <213> Artificial Sequence
<220> primer_bind
   <223> OK20 PCR primer
<400> 16
   aagcttggcg cgccagatct ataacttcgt ataatgtatg ctatacgaag ttatccgaga 60
   ttcatcaact cattgc 76
<210> 17
   <211> 30
   <212> DNA
   <213> Artificial Sequence
<220> primer_bind
   <223> LY7 PCR primer
<400> 17
   cgggatcctg agggagacct aactacatag 30
<210> 18
   <211> 51
   <212> DNA
   <213> Artificial Sequence
<220> primer_bind
   <223> LY8 PCR primer
<400> 18
   tagcgtagat tgtctgatca tggtaccttt tagtttatgt atgtgttttt t 51
<210> 19
   <211> 51
   <212> DNA
   <213> Artificial Sequence
<220> primer_bind
   <223> LY9 PCR primer
<400> 19
   aaaaaacaca tacataaact aaaaggtacc atgatcagac aatctacgct a 51
<210> 20
   <211> 28
   <212> DNA
   <213> Artificial Sequence
<220> primer_bind
   <223> LY10 PCR primer
<400> 20
   gcgtcgacga agcgtcagat cagacaca 28
<210> 21
   <211> 37
   <212> DNA
   <213> Artificial Sequence
<220> primer_bind
   <223> Ko446 PCR primer
<400> 21
   ccgggatcct ctagagtgat gacggtgaaa acctctg 37
<210> 22
   <211> 38
   <212> DNA
   <213> Artificial Sequence
<220> primer_bind
   <223> Ko448 PCR Primer
<400> 22
   ccgggatcct ctagactgag gacataaaat acacaccg 38
<210> 23
   <211> 687
   <212> PRT
   <213> Saccharomyces kudriavzevil
<400> 23
   Met Ile Arg Gln Ser Thr Leu Lys Asn Phe Ala Leu Lys Arg Cys Phe
   1               5                   10                  15
   Gln Gln Ile Ala Tyr Arg Ser Thr Pro Ala Met Arg Ser Val Ala Leu
               20                  25                  30
   Ala Gln Arg Phe Tyr Ser Ser Ser Ser Arg Tyr Tyr Ser Ala Ser Pro
           35                  40                  45
   Leu Pro Val Ser Lys Arg Pro Glu Pro Ala Pro Ser Phe Asn Val Glu
       50                  55                  60
   Pro Leu Glu Gln Ala Pro Glu Pro Ser Lys Leu Ala Lys Arg Leu Arg
   65 70 75 80
   Thr Glu Pro Asp Met Asp Thr Ser Phe Val Gly Leu Thr Gly Gly Gln
   85 90 95
   Ile Phe Asn Glu Met Met Ser Arg Gln Asn Val Asp Thr Val Phe Gly
   100 105 110
   Tyr Pro Gly Gly Ala Ile Leu Pro Val Tyr Asp Ala Ile His Asn Ser
   115 120 125
   Asp Lys Phe Asn Phe Val Leu Pro Lys His Glu Gln Gly Ala Gly His
   130 135 140
   Met Ala Glu Gly Tyr Ala Arg Ala Ser Gly Lys Pro Gly Val Val Leu
   145 150 155 160
   Val Thr Ser Gly Pro Gly Ala Thr Asn Val Val Thr Pro Met Ala Asp
   165 170 175
   Ala Phe Ala Asp Gly Ile Pro Met Val Val Phe Thr Gly Gln Val Pro
   180 185 190
   Thr Ser Ala Ile Gly Thr Asp Ala Phe Gln Glu Ala Asp Val Val Gly
   195 200 205
   Ile Ser Arg Ser Cys Thr Lys Trp Asn Val Met Val Lys Thr Val Glu
   210 215 220
   Glu Leu Pro Leu Arg Ile Asn Glu Ala Phe Glu Ile Ala Thr Ser Gly
   225 230 235 240
   Arg Pro Gly Pro Val Leu Val Asp Leu Pro Lys Asp Val Thr Ala Ala
   245 250 255
   Ile Leu Arg Asn Pro Ile Pro Thr Lys Thr Thr Leu Pro Ser Asn Ala
   260 265 270
   Leu Asn Gln Leu Thr Ser His Ala Gln Asp Glu Phe Val Met Gln Ser
   275 280 285
   Ile Ser Lys Ala Ala Asp Leu Ile Asn Leu Ala Lys Lys Pro Val Leu
   290 295 300
   Tyr Val Gly Ala Gly Ile Leu Asn Asn Ala Asp Gly Pro Arg Leu Leu
   305 310 315 320
   Lys Glu Leu Ser Glu Arg Ala Gln Ile Pro Val Thr Thr Thr Leu Gln
                   325                 330                 335
   Gly Leu Gly Ser Phe Asp Gln Glu Asp Pro Lys Ser Leu Asp Met Leu
               340                 345                 350
   Gly Met His Gly Cys Val Thr Ala Asn Leu Ala Val Gln Asn Ala Asp
           355                 360                 365
   Leu Leu Ile Ala Val Gly Ala Arg Phe Asp Asp Arg Val Thr Cys Asn
       370                 375                 380
   Ile Ala Lys Phe Ala Pro Glu Ala Arg Arg Ala Ala Ala Glu Gly Arg
   385                 390                 395                 400
   Gly Gly Ile Val His Phe Glu Val Val Pro Lys Asn Ile Asn Lys Val
                   405                 410                 415
   Val Glu Thr Gln Ile Ala Val Glu Gly Asp Ala Thr Ser Asn Leu Asp
               420                 425                 430
   Lys Met Met Pro Lys Ile Phe Pro Val Lys Glu Arg Ser Glu Trp Phe
           435                 440                 445
   Gly Gln Ile Asn Lys Trp Lys Lys Glu Phe Pro Tyr Ala Tyr Met Met
       450                 455                 460
   Glu Thr Pro Gly Ser Lys Ile Lys Pro Gln Thr Val Ile Thr Lys Leu
   465                 470                 475                 480
   Ser Lys Ile Ala Asn Asp Thr Gly Arg His Val Ile Val Thr Thr Gly
                   485                 490                 495
   Val Gly Gln His Gln Met Trp Ala Ala Gln His Trp Thr Trp Lys Asn
               500                 505                 510
   Pro Arg Thr Phe Ile Thr Ser Gly Gly Leu Gly Thr Met Gly Tyr Gly
           515                 520                 525
   Leu Pro Ser Ala Ile Gly Ala Gln Val Ala Lys Pro Glu Ser Leu Val
       530                 535                 540
   Ile Asp Ile Asp Gly Asp Ala Ser Phe Asn Met Thr Leu Thr Glu Leu
   545                 550                 555                 560
   Ser Ser Ala Val Gln Ala Gly Thr Pro Val Lys Ile Leu Ile Leu Asn
                   565                 570                 575
   Asn Glu Glu Gln Gly Met Val Thr Gln Trp Gln Ser Leu Phe Tyr Glu
                   580                 585                 590
   His Arg Tyr Ser His Thr His Gln Leu Asn Pro Asp Phe Ile Lys Leu
               595                 600                 605
   Ala Glu Ala Met Gly Leu Lys Gly Leu Arg Val Lys Lys Gln Glu Glu
       610                 615                 620
   Leu Asp Ala Lys Leu Lys Glu Phe Val Ser Thr Lys Gly Pro Val Leu
   625                 630                 635                 640
   Leu Glu Val Glu Val Asp Lys Lys Val Pro Val Leu Pro Met Val Pro
                   645                 650                 655
   Ala Gly Lys Gly Leu Asp Glu Ser Ile Asn Phe Asp Pro Glu Val Glu
               660                 665                 670
   Lys Gln Gln Thr Glu Leu Arg His Lys Arg Thr Gly Gly Lys Tyr
           675                 680                 685
<210> 24
   <211> 684
   <212> PRT
   <213> Naumovozyma castellii
<400> 24
   Met Leu Arg Gln Ala Ala Leu Lys Asn Val Thr Ala Lys Arg Cys Phe
   1               5                   10                  15
   Gln Gln Phe Ala Met Lys Ser Ser Thr Val Ser Val Ala Thr Arg Tyr
               20                  25                  30
   Tyr Arg Ser Thr Pro Arg Phe Tyr Ser Ser Ala Ser Gln Ala Thr Ser
           35                  40                  45
   Val Arg Pro Glu Pro Ala Pro Ser Phe Asn Val Asp Pro Ala Glu Asn
       50                  55                  60
   Ala Thr Ser Lys Pro Ser Lys Leu Ala Lys Lys Ile Arg Gln Glu Pro
   65                  70                  75                  80
   Gln Met Asp Thr Ser Phe Val Gly Leu Ser Gly Gly Gln Ile Phe Asn
                   85                  90                  95
   Glu Met Met Lys Arg Gln Asn Val Asp Thr Val Phe Gly Tyr Pro Gly
               100                 105                 110
   Gly Ala Ile Leu Pro Val Tyr Asp Ala Ile Tyr Asn Ser Glu Ala Phe
           115                 120                 125
   Asn Phe Val Leu Pro Lys His Glu Gln Gly Ala Gly His Met Ala Glu
       130                 135                 140
   Gly Tyr Ala Arg Ala Ser Gly Lys Pro Gly Val Val Leu Val Thr Ser
   145                 150                 155                 160
   Gly Pro Gly Ala Thr Asn Val Val Thr Pro Met Ala Asp Ala Leu Ala
                   165                 170                 175
   Asp Gly Ile Pro Met Val Val Phe Thr Gly Gln Val Pro Thr Ser Ala
               180                 185                 190
   Ile Gly Thr Asp Ala Phe Gln Glu Ala Asp Val Val Gly Ile Ser Arg
           195                 200                 205
   Ser Cys Thr Lys Trp Asn Val Met Val Lys Ser Val Ala Glu Leu Pro
       210                 215                 220
   Leu Arg Ile Asn Glu Ala Phe Glu Ile Ala Met Ser Gly Arg Pro Gly
   225                 230                 235                 240
   Pro Val Leu Val Asp Leu Pro Lys Asp Val Thr Ala Ala Ile Leu Arg
                   245                 250                 255
   Glu Pro Ile Pro Ile Lys Ser Thr Leu Pro Ser Gly Thr Leu Thr Gln
               260                 265                 270
   Ile Lys Ser Ile Ala Gln Glu Asp Phe Leu Ala Asp Ser Ile Asn Arg
           275                 280                 285
   Ala Ala Asp Leu Ile Asn Leu Ala Lys Lys Pro Val Leu Tyr Val Gly
       290                 295                 300
   Asn Gly Ile Phe Asn Asn Glu Asp Gly Pro Arg Leu Leu Lys Glu Leu
   305                 310                 315                 320
   Ser Glu Arg Ala Gln Ile Pro Val Thr Thr Thr Ile Gln Gly Leu Gly
                   325                 330                 335
   Ala Phe Asp Gln Glu Asp Pro Lys Ser Leu Asp Met Leu Gly Met His
               340                 345                 350
   Gly Cys Ala Thr Ala Asn Leu Ala Met Gln Asn Ala Asp Leu Ile Ile
           355                 360                 365
   Ala Val Gly Ala Arg Phe Asp Asp Arg Val Thr Gly Asn Ile Ala Lys
       370                 375                 380
   Phe Ala Pro Glu Ala Arg Arg Ala Ala Asn Glu Gly Arg Gly Gly Ile
   385                 390                 395                 400
   Ile His Phe Glu Ile Ser Pro Lys Asn Ile Asn Lys Val Val Glu Ala
                   405                 410                 415
   Gln Val Ala Val Glu Gly Asp Ala Ala Ala Asn Ile Asp Lys Met Leu
               420                 425                 430
   Pro Ala Ile Phe Pro Ile Lys Glu Arg Thr Glu Trp Phe Lys Glu Ile
           435                 440                 445
   Asn Gln Trp Lys Lys Asp Tyr Pro Tyr Asp Tyr Met Lys Glu Val Pro
       450                 455                 460
   Gly Gly Lys Leu Lys Pro Gln Thr Val Ile Ser Lys Leu Ser Lys Val
   465                 470                 475                 480
   Ala Asn Ala Thr Gly Arg Pro Val Ile Val Thr Thr Gly Val Gly Gln
                   485                 490                 495
   His Gln Met Trp Thr Ala Gln His Trp Thr Trp Lys His Pro Arg Ser
               500                 505                 510
   Phe Ile Thr Ser Gly Gly Leu Gly Thr Met Gly Tyr Gly Leu Pro Ser
           515                 520                 525
   Ala Ile Gly Ala Gln Val Ala His Pro Glu Ala Leu Val Ile Asp Ile
       530                 535                 540
   Asp Gly Asp Ala Ser Phe Asn Met Thr Leu Thr Glu Leu Ser Ser Ala
   545                 550                 555                 560
   Val Gln Ala Gly Thr Pro Val Lys Ile Met Ile Leu Asn Asn Glu Glu
                   565                 570                 575
   Gln Gly Met Val Thr Gln Trp Gln Ser Leu Phe Tyr Glu Asn Arg Tyr
               580                 585                 590
   Ser His Thr His Gln Leu Asn Pro Asp Phe Met Lys Leu Ala Asp Ala
           595                 600                 605
   Met Gly Leu Lys Gly Ile Arg Val Lys Lys Gln Glu Glu Leu Asp Gly
               610                 615                 620
   Ala Leu Lys Glu Phe Val Ser Thr Pro Gly Pro Val Leu Leu Glu Val
   625                 630                 635                 640
   Glu Val Glu Lys Lys Val Pro Val Leu Pro Met Val Pro Ala Gly Lys
                   645                 650                 655
   Gly Leu Asp Glu Phe Met Ser Tyr Asp Pro Lys Val Glu Lys Glu Gln
               660                 665                 670
   Asn Glu Leu Arg His Lys Arg Thr Gly Gly Lys His
           675                 680
<210> 25
   <211> 685
   <212> PRT
   <213> Naumovozyma dairenensis
<400> 25
   Met Leu Arg Gln Ala Ala Leu Lys Asn Val Ala Thr Lys Arg Gly Phe
   1               5                   10                  15
   Gln Gln Leu Thr Leu Arg Ser Ser Lys Ala Pro Ser Ser Ile Ala Phe
               20                  25                  30
   Arg Tyr Tyr Lys Ser Ile Pro Arg Asn Tyr Ser Ser Val Ser Asn Ala
           35                  40                  45
   Ala Ala Val Arg Pro Glu Pro Ala Pro Ser Phe Asn Val Asp Pro Ala
       50                  55                  60
   Ser Gln Pro Ser Gln Lys Gln Pro Val Lys Leu Ser Lys Lys Leu Arg
   65                  70                  75                  80
   Thr Glu Pro Glu Met Asp Ser Ser Phe Val Gly Leu Ser Gly Gly Gln
                   85                  90                  95
   Ile Phe Asn Glu Met Met Lys Arg Gln Asn Val Asp Thr Val Phe Gly
               100                 105                 110
   Tyr Pro Gly Gly Ala Ile Leu Pro Val Tyr Asp Ala Ile Tyr Asn Ser
           115                 120                 125
   Asp Ala Phe Lys Phe Val Leu Pro Lys His Glu Gln Gly Ala Gly His
       130                 135                 140
   Met Ala Glu Gly Tyr Ala Arg Ala Ser Gly Lys Thr Gly Val Val Leu
   145 150 155 160
   Val Thr Ser Gly Pro Gly Ala Thr Asn Val Val Thr Pro Met Ala Asp
   165 170 175
   Ala Leu Ala Asp Gly Ile Pro Met Val Val Phe Thr Gly Gln Val Pro
   180 185 190
   Thr Ser Ala Ile Gly Thr Asp Ala Phe Gln Glu Ala Asp Val Val Gly
   195 200 205
   Ile Ser Arg Ser Cys Thr Lys Trp Asn Val Met Val Arg Ser Val Ala
   210 215 220
   Glu Leu Pro Leu Arg Ile Asn Glu Ala Phe Glu Ile Ala Asn Ser Gly
   225 230 235 240
   Arg Pro Gly Pro Val Leu Val Asp Leu Pro Lys Asp Val Thr Ala Ala
   245 250 255
   Ile Leu Arg Asn Pro Ile Pro Ile Lys Ser Thr Leu Pro Ile Thr Ser
   260 265 270
   Gln Leu Lys Ser Val Ala Glu Glu Ala Tyr Thr Met Glu Ser Ile Asn
   275 280 285
   Arg Ala Ala Asp Leu Ile Asn Leu Ala Lys Arg Pro Val Leu Tyr Val
   290 295 300
   Gly Asn Gly Ile Met Asn Asn Pro Asp Gly Pro Arg Leu Leu Lys Glu
   305 310 315 320
   Leu Ser Asp Arg Ala Gln Ile Pro Val Thr Thr Thr Ile Gln Gly Leu
   325 330 335
   Gly Ala Phe Asp Gln Glu Asp Pro Lys Ser Leu Asp Met Leu Gly Met
   340 345 350
   His Gly Cys Ala Thr Ala Asn Leu Ala Met Gln Asn Ala Asp Leu Val
   355 360 365
   Ile Ala Leu Gly Ala Arg Phe Asp Asp Arg Val Thr Gly Asn Ile Ala
   370 375 380
   Lys Phe Ala Pro Glu Ala Arg Arg Ala Ala Leu Glu Glu Arg Gly Gly
   385 390 395 400
   Ile Ile His Phe Glu Ile Ser Pro Lys Asn Ile Asn Lys Val Val Glu
                   405                 410                 415
   Ala Gln Val Ala Val Glu Gly Asp Ala Ala Ser Asn Val Ser Lys Leu
               420                 425                 430
   Leu Pro Leu Val Phe Pro Val Lys Glu Arg Lys Asp Trp Phe Lys Gln
           435                 440                 445
   Ile Asn Gln Trp Lys Lys Glu Tyr Pro Tyr Ser Tyr Met Lys Glu Thr
       450                 455                 460
   Ala Glu Ser Lys Ile Lys Pro Gln Thr Val Ile Ser Lys Leu Ser Lys
   465                 470                 475                 480
   Ile Ala Asn Gly Thr Gly Lys Pro Val Val Val Thr Thr Gly Val Gly
                   485                 490                 495
   Gln His Gln Met Trp Ala Ala Gln His Trp Thr Trp Lys His Pro Arg
               500                 505                 510
   Ser Phe Ile Thr Ser Gly Gly Leu Gly Thr Met Gly Tyr Gly Leu Pro
           515                 520                 525
   Ala Ala Ile Gly Ala Gln Val Ala Arg Pro Asp Ala Ile Val Ile Asp
       530                 535                 540
   Ile Asp Gly Asp Ala Ser Phe Asn Met Thr Leu Thr Glu Leu Ser Ser
   545                 550                 555                 560
   Ala Val Gln Ala Gly Val Pro Ile Lys Ile Leu Leu Leu Asn Asn Glu
                   565                 570                 575
   Glu Gln Gly Met Val Thr Gln Trp Gln Ser Leu Phe Tyr Glu His Arg
               580                 585                 590
   Tyr Ser His Thr His Gln Leu Asn Pro Asp Phe Met Lys Leu Ala Asp
           595                 600                 605
   Ala Met Gly Leu Lys Gly Ile Arg Val Lys Asn Gln Gly Glu Leu Asp
       610                 615                 620
   Gly Ala Leu Lys Glu Phe Val Asp Tyr Gln Gly Pro Val Leu Leu Glu
   625                 630                 635                 640
   Val Glu Val Glu Lys Lys Val Pro Val Leu Pro Met Val Pro Ala Gly
                   645                 650                 655
   Lys Gly Leu Asp Glu Phe Ile Asn Phe Asp Pro Glu Met Glu Lys Glu
   660 665 670
   Gln Asn Glu Leu Arg His Lys Arg Thr Gly Gly Lys His
   675 680 685
<210> 26
   <211> 677
   <212> PRT
   <213> Candida glabrata
<400> 26
   Met Thr Arg Arg Gly Leu Thr Asp Pro Met Ile Arg Ile Ser Gly Val
   1 5 10 15
   Val Ala Arg Ala Cys Arg Arg Ser Ala Ala Tyr Gln Val Cys Lys Tyr
   20 25 30
   Ser Val Arg Ala Val Pro Arg Pro Glu Pro Ser Pro Ser Phe Asn Val
   35 40 45
   Asp Pro Asp Thr Ala Ala Ala Asn Thr Gly Ser Gly Val Lys Leu Ser
   50 55 60
   Ser Lys Leu Lys Asn Val Ala Thr Gln Lys Met Asp Asn Ser Phe Val
   65 70 75 80
   Gly Leu Thr Gly Gly Gln Ile Phe Asn Glu Met Met Ala Arg Asn Asn
   85 90 95
   Val Asp Thr Val Phe Gly Tyr Pro Gly Gly Ala Ile Leu Pro Val Tyr
   100 105 110
   Asp Ala Ile His Asn Ser Asp Lys Phe Lys Phe Val Leu Pro Lys His
   115 120 125
   Glu Gln Gly Ala Gly His Met Ala Glu Gly Tyr Ala Arg Ala Ser Gly
   130 135 140
   Lys Pro Gly Val Val Leu Val Thr Ser Gly Pro Gly Ala Thr Asn Val
   145 150 155 160
   Val Thr Pro Met Ala Asp Ala Leu Ala Asp Gly Ile Pro Met Val Val
   165 170 175
   Phe Thr Gly Gln Val Pro Thr Thr Ala Ile Gly Thr Asp Ala Phe Gln
   180 185 190
   Glu Ala Asp Ile Val Gly Ile Ser Arg Ser Cys Thr Lys Trp Asn Val
   195 200 205
   Met Val Lys Asn Val Ala Glu Leu Pro Leu Arg Ile Asn Glu Ala Phe
   210 215 220
   Gln Ile Ala Thr Ser Gly Arg Pro Gly Pro Val Leu Val Asp Leu Pro
   225 230 235 240
   Lys Asp Val Thr Ala Ala Ile Leu Arg Thr Pro Ile Pro Val Lys Ser
   245 250 255
   Thr Leu Pro Ser Thr Ala Leu Gln Gln Leu Thr Ala Gly Val Gln Asp
   260 265 270
   Gln Phe Val Ile Glu Asn Ile Lys Lys Ser Ala Asp Leu Ile Asn Ile
   275 280 285
   Ala Lys Asn Pro Val Leu Tyr Val Gly Gly Gly Ile Leu Asn Asn Val
   290 295 300
   Asp Gly Pro Arg Leu Val Lys Glu Leu Ser Glu Arg Ala Gln Ile Pro
   305 310 315 320
   Val Thr Thr Thr Leu Gln Gly Leu Gly Ala Phe Asp Gln Glu Asp Pro
   325 330 335
   Lys Ser Leu Asp Met Leu Gly Met His Gly Cys Ala Thr Ala Asn Leu
   340 345 350
   Ala Val Gln Asn Ser Asp Leu Ile Ile Ala Val Gly Ala Arg Phe Asp
   355 360 365
   Asp Arg Val Thr Gly Asn Ile Thr Lys Phe Ala Pro Glu Ala Arg Lys
   370 375 380
   Ala Ala Leu Glu Gly Arg Gly Gly Ile Ile His Phe Glu Ile Thr Pro
   385 390 395 400
   Lys Asn Ile Asn Lys Val Val Glu Ala Gln Val Ala Val Glu Gly Asp
   405 410 415
   Ala Ala Ser Asn Leu Ser Lys Leu Ile Pro Leu Val Phe Pro Val Lys
   420 425 430
   Glu Arg Pro Glu Trp Phe Glu Lys Ile Asn Lys Trp Lys Lys Glu Phe
   435 440 445
   Pro Tyr Ser Tyr Ser Leu Glu Thr Pro Gly Ser Arg Ile Lys Pro Gln
   450 455 460
   Thr Ala Ile Ala Lys Leu Ser Lys Ile Ala Asn Ala Thr Ser Lys Glu
   465 470 475 480
   Val Ile Val Thr Thr Gly Val Gly Gln His Gln Met Trp Ala Ala Gln
   485 490 495
   His Trp Thr Trp Lys Asn Pro Arg Thr Phe Ile Thr Ser Gly Gly Leu
   500 505 510
   Gly Thr Met Gly Tyr Gly Leu Pro Ser Ala Ile Gly Ala Gln Val Ala
   515 520 525
   Lys Pro Asp Ala Leu Val Ile Asp Ile Asp Gly Asp Ala Ser Phe Asn
   530 535 540
   Met Ser Leu Gln Glu Leu Ser Ser Ala Val Gln Ala Asn Ala Pro Val
   545 550 555 560
   Lys Ile Leu Val Leu Asn Asn Glu Glu Gln Gly Met Val Thr Gln Trp
   565 570 575
   Gln Ser Leu Phe Tyr Glu His Arg Tyr Ser His Thr His Gln Leu Asn
   580 585 590
   Pro Asp Phe Val Lys Leu Ala Glu Ala Met Gly Met Lys Gly Met Arg
   595 600 605
   Val Lys Asp Gln Ala Glu Leu Glu Lys Thr Leu Lys Glu Phe Val Asp
   610 615 620
   Tyr Gln Gly Pro Val Leu Leu Glu Val Glu Val Glu Lys Lys Val Pro
   625 630 635 640
   Val Leu Pro Met Val Pro Ala Gly Lys Gly Leu His Glu Phe Ile Asn
   645 650 655
   Tyr Asp Pro Glu Met Glu Arg Glu Gln Asn Glu Leu Arg His Lys Arg
   660 665 670
   Thr Gly Gly Lys His
   675
<210> 27
   <211> 682
   <212> PRT
   <213> Torulaspora delbrueckii
<400> 27
   Met Ile Arg Gln Ala Thr Ile Lys Gln Phe Ala Ala Lys Arg Cys Phe
   1               5                   10                  15
   Arg Glu Ile Ala Ser Arg Ser Ala Val Tyr Gly Thr Val Arg Tyr Tyr
               20                  25                  30
   Gly Ser Ala Ser Arg Asn Tyr Ser Ala Thr Val Ala Ser Ala Val Ala
           35                  40                  45
   Ser Gly Arg Pro Glu Pro Ala Pro Ser Phe Asn Val Asp Pro Ala Ala
       50                  55                  60
   Gly Lys Pro Ser Lys Leu Asn Gln Lys Leu Arg Thr Asp Pro Gln Met
   65                  70                  75                  80
   Asp Ser Ser Phe Ile Gly Leu Thr Gly Gly Gln Ile Phe His Glu Met
                   85                  90                  95
   Met Ile Arg His Asn Val Asp Thr Val Phe Gly Tyr Pro Gly Gly Ala
               100                 105                 110
   Ile Leu Pro Val Tyr Asp Ala Ile His Asp Ser Lys Ser Phe Asn Phe
           115                 120                 125
   Val Leu Pro Arg His Glu Gln Gly Ala Gly His Met Ala Glu Gly Tyr
       130                 135                 140
   Ala Arg Ala Ser Gly Lys Pro Gly Val Val Leu Val Thr Ser Gly Pro
   145                 150                 155                 160
   Gly Ala Thr Asn Val Val Thr Pro Met Ala Asp Ala Leu Ala Asp Gly
                   165                 170                 175
   Val Pro Met Val Val Phe Thr Gly Gln Val Pro Thr Ser Ala Ile Gly
               180                 185                 190
   Thr Asp Ala Phe Gln Glu Ala Asp Val Val Gly Ile Ser Arg Ser Cys
           195                 200                 205
   Thr Lys Trp Asn Val Met Val Arg Ser Ile Glu Glu Leu Pro Leu Arg
       210                 215                 220
   Ile Asn Glu Ala Phe Glu Ile Ala Val Ser Gly Arg Pro Gly Pro Val
   225 230 235 240
   Leu Val Asp Leu Pro Lys Asp Val Thr Ala Ala Ile Leu Lys Asn Pro
   245 250 255
   Ile Pro Thr Lys Thr Thr Leu Pro Ser Asn Ser Leu Ala Gln Leu Thr
   260 265 270
   Lys Arg Ala Met Asp Glu Ser Thr Asn Glu Asn Ile Val Arg Thr Ala
   275 280 285
   Asp Leu Leu Asn Lys Ala Lys Thr Pro Val Leu Tyr Val Gly Ala Gly
   290 295 300
   Ile Leu Asn Asn Pro Ala Gly Pro Lys Leu Leu Lys Glu Leu Ser Glu
   305 310 315 320
   Arg Ala Gln Ile Pro Val Thr Thr Thr Ile Gln Ala Leu Gly Ala Phe
   325 330 335
   Asp Gln Gln Asp Pro Lys Ser Leu Asp Met Leu Gly Met His Gly Cys
   340 345 350
   Ala Thr Ala Asn Met Ala Ile Gln Asn Ala Asp Leu Ile Leu Ala Val
   355 360 365
   Gly Ser Arg Phe Asp Asp Arg Val Thr Leu Asn Ile Ser Lys Phe Ala
   370 375 380
   Pro Glu Ala Arg Arg Ala Ala Leu Glu Asn Arg Gly Gly Ile Val His
   385 390 395 400
   Phe Glu Ile Cys Pro Lys Asn Ile Ser Lys Val Val Glu Thr Gln Val
   405 410 415
   Ala Val Glu Gly Asp Ala Ala Ser Asn Ile Glu Lys Leu Leu Pro Leu
   420 425 430
   Val Val Pro Val Lys Glu Arg Thr Glu Trp Phe Ala Gln Ile Glu Glu
   435 440 445
   Trp Lys Glu Lys Tyr Pro Tyr Ala Tyr Gln Lys Glu Thr Pro Gly Ser
   450 455 460
   Lys Ile Lys Pro Gln Thr Val Ile Ser Lys Leu Ser Asp Leu Ala Asn
   465 470 475 480
   Lys Ser Asp Lys Glu Val Ile Val Thr Thr Gly Val Gly Gln His Gln
   485 490 495
   Met Trp Ala Ala Gln Tyr Trp Thr Trp Lys Ser Pro Arg Ser Phe Ile
   500 505 510
   Thr Ser Gly Gly Leu Gly Thr Met Gly Tyr Gly Leu Pro Ala Ala Ile
   515 520 525
   Gly Ala Gln Val Ala Lys Pro Asp Ala Leu Val Ile Asp Ile Asp Gly
   530 535 540
   Asp Ala Ser Phe Asn Met Thr Leu Met Glu Leu Ser Ser Ala Val Gln
   545 550 555 560
   Ala Lys Thr Pro Val Lys Ile Leu Leu Leu Asn Asn Glu Glu Gln Gly
   565 570 575
   Met Val Thr Gln Trp Gln Ser Leu Phe Tyr Glu His Arg Tyr Ser His
   580 585 590
   Thr His Gln Leu Asn Pro Asp Phe Leu Lys Leu Ala Asp Ala Met Gly
   595 600 605
   Leu Lys Gly Leu Arg Leu Ser Lys Gln Glu Asp Val Asp Ala Thr Leu
   610 615 620
   Lys Glu Phe Ile Glu Thr Glu Gly Pro Val Leu Leu Glu Val Lys Val
   625 630 635 640
   Glu Pro Lys Val Pro Val Leu Pro Met Val Pro Ala Gly Lys Gly Leu
   645 650 655
   Asp Glu Phe Ile Val Phe Asp Pro Lys Val Glu Met Glu Gln Asn Ala
   660 665 670
   Leu Arg His Lys Arg Thr Gly Gly Lys His
   675 680
<210> 28
   <211> 682
   <212> PRT
   <213> Kazachstania africana
<400> 28
   Met Met Leu Arg Cys Pro Val Leu Arg Ser His Ala Leu Lys Arg Cys
   1 5 10 15
   Ala Arg Ser Lys Ser Pro Phe Leu Ala Leu Arg Tyr Tyr Thr Asn Ala
               20                  25                  30
   Thr Lys Thr Leu Asn Ser Ala Ser Ala Gln Ala Val Ala Thr Asn Arg
           35                  40                  45
   Arg Pro Glu Pro Ala Pro Ser Phe Asp Val Asp Pro Leu Ser Ser Glu
       50                  55                  60
   Ile Arg Glu Ser Phe Gly Lys Lys Ala Lys Val Asp Pro Met Lys Met
   65                  70                  75                  80
   Asp Asn Ser Leu Val Gly Leu Thr Gly Gly Gln Ile Phe Asn Glu Met
                   85                  90                  95
   Met Ala Arg His Asn Val Asp Thr Val Phe Gly Tyr Pro Gly Gly Ala
               100                 105                 110
   Ile Leu Pro Val Tyr Asp Ala Ile Tyr Gln Ser Lys Asn Phe Lys Phe
           115                 120                 125
   Val Leu Pro Lys His Glu Gln Gly Ala Gly His Met Ala Glu Gly Tyr
       130                 135                 140
   Ala Arg Ala Ser Gly Lys Pro Gly Ile Val Leu Val Thr Ser Gly Pro
   145                 150                 155                 160
   Gly Ala Thr Asn Val Val Thr Pro Met Ala Asp Ala Leu Ala Asp Gly
                   165                 170                 175
   Ile Pro Met Val Val Phe Thr Gly Gln Val Pro Thr Thr Ala Ile Gly
               180                 185                 190
   Thr Asp Ala Phe Gln Glu Ala Asp Val Val Gly Ile Ser Arg Ser Cys
           195                 200                 205
   Thr Lys Trp Asn Val Met Ile Lys Asn Val Ala Glu Leu Pro Val Arg
       210                 215                 220
   Ile Asn Glu Ala Phe Glu Ile Ala Thr Ser Gly Arg Pro Gly Pro Val
   225                 230                 235                 240
   Leu Val Asp Leu Pro Lys Asp Val Thr Ala Ala Ile Leu Arg Asp Ala
                   245                 250                 255
   Ile Pro Val Gly Ser Ser Leu Pro Ser Asn Ala Leu Ser Gln Leu Thr
               260                 265                 270
   Asn Lys Ala Gln Asp Glu Phe Val Leu Asp Asn Ile Asn Lys Ala Ala
   275 280 285
   Asp Leu Ile Asn Leu Ala Lys Asn Pro Val Leu Tyr Val Gly Gly Gly
   290 295 300
   Ile Leu Gly Asn Glu Asp Gly Pro Lys Leu Leu Lys Gln Leu Ser Glu
   305 310 315 320
   Arg Ala Gln Ile Pro Val Thr Thr Ser Leu Leu Gly Leu Gly Ala Phe
   325 330 335
   Asp Gln Glu Asp Pro Lys Ser Leu Asp Met Leu Gly Met His Gly Cys
   340 345 350
   Ala Thr Ala Asn Leu Ala Met Gln Asn Ala Asp Leu Ile Ile Ala Ile
   355 360 365
   Gly Thr Arg Phe Asp Asp Arg Val Thr Gly Asn Ile Ala Lys Phe Ala
   370 375 380
   Pro Glu Ala Arg Arg Ala Ala Leu Glu Asn Arg Gly Gly Ile Ile His
   385 390 395 400
   Phe Glu Ile Ser Pro Lys Asn Ile Asn Lys Val Val Glu Ala Gln Ile
   405 410 415
   Ala Val Glu Gly Asp Ala Thr Ala Asn Leu Gln Ser Ile Leu Pro Lys
   420 425 430
   Ile Phe Pro Val Lys Glu Arg Thr Glu Trp Phe Ala Lys Ile Lys Glu
   435 440 445
   Trp Lys Thr Lys Tyr Pro Tyr Ser Tyr Met Lys Glu Thr Pro Asn Ser
   450 455 460
   Lys Leu Lys Pro Gln Thr Val Ile Ser Lys Leu Ser Lys Ile Ala Asn
   465 470 475 480
   Ala Thr Gly Lys Glu Val Ile Val Thr Thr Gly Val Gly Gln His Gln
   485 490 495
   Met Trp Ala Ala Gln His Trp Thr Trp Lys His Pro Arg Thr Phe Ile
   500 505 510
   Thr Ser Gly Gly Leu Gly Thr Met Gly Tyr Gly Leu Pro Ala Ala Ile
   515 520 525
   Gly Ala Gln Val Ala Lys Pro Asp Ala Leu Val Ile Asp Ile Asp Gly
       530                 535                 540
   Asp Ala Ser Phe Asn Met Thr Leu Thr Glu Leu Ser Ser Ala Val Gln
   545                 550                 555                 560
   Ala Gly Ala Pro Val Lys Ile Leu Leu Leu Asn Asn Glu Glu Gln Gly
                   565                 570                 575
   Met Val Thr Gln Trp Gln Ser Leu Phe Tyr Glu Asn Arg Tyr Ser His
               580                 585                 590
   Thr His Gln Leu Asn Pro Asp Phe Ile Lys Leu Ala Glu Ala Met Gly
           595                 600                 605
   Leu Lys Ala Ile Lys Val Ser Lys Gln Asn Gln Leu Asp Lys Ala Leu
       610                 615                 620
   Gln Glu Phe Val Glu Tyr Asp Gly Pro Val Leu Leu Glu Val Glu Val
   625                 630                 635                 640
   Glu Lys Lys Val Pro Val Leu Pro Met Val Pro Ala Gly Lys Gly Leu
                   645                 650                 655
   His Glu Phe Met Ser Phe Asp Glu Asn Thr Glu Lys Glu Gln Asn Glu
               660                 665                 670
   Leu Arg Arg Asn Arg Thr Asn Gly Lys Tyr
           675                 680
<210> 29
   <211> 693
   <212> PRT
   <213> Kazachstania naganishii
<400> 29
   Met Leu Arg Ser Gln Leu Val Lys Asn Lys Ala Leu His Arg Ala Thr
   1               5                   10                  15
   Ala Arg Gly Tyr Ser Gln Asn Val Trp Thr Arg Phe Val Arg Ser Ala
               20                  25                  30
   Pro Ala Thr Cys Arg Tyr Thr Ala Val Arg Cys Ala Ser Arg Ser Ser
           35                  40                  45
   Ser Ala Arg Pro Glu Pro Ser Pro Ser Phe Asp Leu Asp Pro Ala Thr
       50                  55                  60
   Ser Leu Tyr Lys Ala Ser Asn Glu Ser Ser Ile Lys Arg Ser Lys Leu
   65                  70                  75                  80
   Ser Asp Lys Leu Arg Ser Ala Thr Glu Leu Asp Asn Ser Leu Val Gly
                   85                  90                  95
   Leu Thr Gly Gly Gln Ile Phe Asn Glu Met Met Ser Arg His Asn Val
               100                 105                 110
   Asp Thr Val Phe Gly Tyr Pro Gly Gly Ala Ile Leu Pro Val Tyr Asp
           115                 120                 125
   Ala Ile Tyr Gly Ser Glu Asn Phe Lys Phe Val Leu Pro Lys His Glu
       130                 135                 140
   Gln Gly Ala Gly His Met Ala Glu Gly Tyr Ala Arg Ala Ser Gly Lys
   145                 150                 155                 160
   Pro Gly Val Val Leu Val Thr Ser Gly Pro Gly Ala Thr Asn Val Val
                   165                 170                 175
   Thr Pro Met Ala Asp Ala Leu Ala Asp Gly Val Pro Met Val Val Phe
               180                 185                 190
   Thr Gly Gln Val Pro Thr Asn Ala Ile Gly Thr Asp Ala Phe Gln Glu
           195                 200                 205
   Ala Asp Val Val Gly Ile Ser Arg Ser Cys Thr Lys Trp Asn Val Met
       210                 215                 220
   Val Lys Asn Val Ala Glu Leu Pro Lys Arg Ile Asn Glu Ala Phe Glu
   225                 230                 235                 240
   Ile Ala Met Ser Gly Arg Pro Gly Pro Val Leu Val Asp Leu Pro Lys
                   245                 250                 255
   Asp Val Thr Ala Ala Val Leu Arg Glu Ala Ile Pro Thr Glu Ser Thr
               260                 265                 270
   Leu Pro Ser Leu Asn Gly Gly Ala Thr Thr Gly Ala Thr Val Phe Asp
           275                 280                 285
   Glu Phe Leu Ala Pro Ala Ile Asn Lys Ala Ala Asp Leu Ile Asn Leu
       290                 295                 300
   Ala Lys Lys Pro Val Leu Tyr Val Gly Asn Gly Ile Leu Asn Asn Glu
   305 310 315 320
   Asp Gly Pro Arg Leu Leu Lys Glu Leu Ser Asp Arg Ala Gln Ile Pro
   325 330 335
   Val Thr Thr Thr Leu Gln Gly Leu Gly Ala Phe Asp Gln Glu Asp Pro
   340 345 350
   Lys Ser Leu Asp Met Leu Gly Met His Gly Cys Ala Thr Ala Asn Leu
   355 360 365
   Ala Ile Gln Asn Ala Asp Leu Ile Ile Ala Val Gly Ala Arg Phe Asp
   370 375 380
   Asp Arg Val Thr Gly Asn Ile Thr Lys Phe Ala Pro Glu Ala Lys Ile
   385 390 395 400
   Ala Ala Gln Glu Asn Arg Gly Gly Ile Ile His Phe Glu Val Ser Pro
   405 410 415
   Lys Asn Ile Asn Lys Val Val Glu Ala Gln Val Ala Val Glu Gly Asp
   420 425 430
   Ala Thr Glu Asn Ile Ala Thr Met Leu Pro Lys Val Phe Pro Val Lys
   435 440 445
   His Arg Ala Glu Trp Phe Lys Gln Ile Glu Gln Trp Lys Gln Lys Phe
   450 455 460
   Pro Tyr Ala Tyr Met Lys Glu Thr Pro Gly Ser Lys Ile Met Pro Gln
   465 470 475 480
   Thr Val Ile Ala Lys Leu Ser Lys Leu Ala Asn Ala Ser Gly Arg Asp
   485 490 495
   Val Val Val Thr Thr Gly Val Gly Gln His Gln Met Trp Ala Ala Gln
   500 505 510
   His Trp Thr Trp Lys Lys Pro Arg Thr Phe Val Thr Ser Gly Gly Leu
   515 520 525
   Gly Thr Met Gly Phe Gly Leu Pro Ala Ala Ile Gly Ala Gln Val Ala
   530 535 540
   Lys Pro Asp Ala Met Val Ile Asp Ile Asp Gly Asp Ala Ser Phe Asn
   545 550 555 560
   Met Thr Leu Thr Glu Leu Ser Ser Ala Val Gln Ala Gly Thr Pro Val
                   565                 570                 575
   Lys Ile Leu Leu Leu Asn Asn Glu Glu Gln Gly Met Val Thr Gln Trp
               580                 585                 590
   Gln Ser Leu Phe Tyr Glu Asn Arg Tyr Ser His Thr His Gln Arg Asn
           595                 600                 605
   Pro Asp Phe Gln Lys Leu Cys Glu Ala Met Gly Val Lys Pro Leu Lys
       610                 615                 620
   Ala Ser Arg Gln Glu Glu Leu Asp Ser Ala Leu Glu Gln Phe Val Glu
   625                 630                 635                 640
   Ala Glu Gly Pro Val Leu Leu Glu Val Ile Val Glu Lys Lys Val Pro
                   645                 650                 655
   Val Leu Pro Met Val Pro Ala Gly Lys Gly Leu Asp Glu Phe Ile Asn
               660                 665                 670
   Phe Asp Pro Glu Glu Glu Lys Lys Gln Asn Glu Leu Arg His Lys Arg
           675                 680                 685
   Thr Lys Gly Lys His
       690
<210> 30
   <211> 671
   <212> PRT
   <213> Vanderwaltozyma polyspora
<400> 30
   Met Leu Arg Thr Thr Ser Ala Pro Val Val Ala Arg Arg Ser Leu Gln
   1               5                   10                  15
   Asn Val Leu Lys Arg Ser Ala Pro Leu Ser Leu Thr Arg Pro Leu Ser
               20                  25                  30
   Tyr Cys Arg Val Arg Leu Ser Gln Ala Gln Glu Arg Pro Glu Pro Ala
           35                  40                  45
   Pro Ser Phe Asn Val Gly Pro Thr Gln Gln Thr Val Phe Pro Lys Lys
       50                  55                  60
   Asp Gln Leu Lys Met Asp Lys Ser Leu Ile Gly Leu Thr Gly Gly Glu
   65                  70                  75                  80
   Ile Phe His Glu Met Met Ile Arg Asn Asn Val Asp Thr Val Phe Gly
                   85                  90                  95
   Tyr Pro Gly Gly Ala Ile Leu Pro Val Tyr Asp Ala Ile Tyr Asn Ser
               100                 105                 110
   Asp Lys Phe Lys Phe Val Leu Pro Arg His Glu Gln Gly Ala Gly His
           115                 120                 125
   Met Ala Glu Gly Tyr Ser Arg Ala Ser Gly Lys Pro Gly Val Val Leu
       130                 135                 140
   Val Thr Ser Gly Pro Gly Ala Thr Asn Val Val Thr Pro Met Ala Asp
   145                 150                 155                 160
   Ala Leu Ala Asp Gly Ile Pro Met Val Val Phe Thr Gly Gln Val Pro
                   165                 170                 175
   Thr Ser Ala Ile Gly Thr Asp Ala Phe Gln Glu Ala Asp Val Ile Gly
               180                 185                 190
   Ile Ser Arg Ser Cys Thr Lys Trp Asn Val Met Val Lys Ser Val Glu
           195                 200                 205
   Glu Leu Pro Arg Arg Ile Asn Glu Ala Phe Glu Ile Ala Thr Ser Gly
       210                 215                 220
   Arg Pro Gly Pro Val Leu Val Asp Leu Pro Lys Asp Val Thr Ala Ala
   225                 230                 235                 240
   Ile Leu Arg Asn Pro Ile Pro Val Asn Ser Thr Leu Pro Ser Asn Ala
                   245                 250                 255
   Leu Asn Gln Leu Val Lys Asn Ala His Asn Glu Phe Thr Glu Glu Ser
               260                 265                 270
   Leu Ser Lys Ala Ala Ser Leu Ile Asn Ile Ala Lys Arg Pro Val Leu
           275                 280                 285
   Tyr Val Gly Gly Gly Ile Leu Asn His Glu Glu Gly Pro Arg Leu Leu
       290                 295                 300
   Arg Glu Leu Ser Glu Arg Ala Gln Ile Pro Val Thr Thr Ser Leu Leu
   305                 310                 315                 320
   Gly Leu Gly Ala Phe Asp Gln Glu Asp Pro Lys Ser Leu Asp Met Leu
                   325                 330                 335
   Gly Met His Gly His Ala Pro Ala Asn Leu Ala Ile Gln Asn Ala Asp
               340                 345                 350
   Leu Ile Ile Ala Val Gly Ala Arg Phe Asp Asp Arg Val Thr Gly Asn
           355                 360                 365
   Ile Ala Lys Phe Ala Pro Glu Ala Arg Arg Ala Ala Leu Glu Asn Arg
       370                 375                 380
   Gly Gly Ile Ile His Phe Glu Ile Thr Ala Lys Asn Ile Asn Lys Val
   385                 390                 395                 400
   Val Asp Thr Gln Val Ala Val Glu Gly Asp Ala Ala Ala Asn Leu Glu
                   405                 410                 415
   Lys Leu Leu Pro Asn Ile Phe Ala Val Lys Glu Arg Lys Asp Trp Phe
               420                 425                 430
   Asp Leu Ile Glu Asp Trp Lys Gln Lys Tyr Pro Tyr Ser Tyr Met Lys
           435                 440                 445
   Glu Glu Pro Gly Ser Arg Ile Lys Pro Gln Thr Ile Ile Ala Lys Leu
       450                 455                 460
   Ser Lys Leu Ala Asn Glu Ser Asn Arg Glu Thr Ile Val Thr Thr Gly
   465                 470                 475                 480
   Val Gly Gln His Gln Met Trp Ala Ala Gln His Trp Thr Trp Lys His
                   485                 490                 495
   Pro Arg Thr Phe Ile Thr Ser Gly Gly Leu Gly Thr Met Gly Phe Gly
               500                 505                 510
   Leu Pro Ser Ala Ile Gly Ala Gln Val Ala Lys Pro Asp Ser Met Val
           515                 520                 525
   Ile Asp Ile Asp Gly Asp Ala Ser Phe Asn Met Thr Leu Met Glu Met
       530                 535                 540
   Thr Ser Ala Val Gln Ala Ser Ala Pro Val Lys Val Leu Leu Leu Asn
   545                 550                 555                 560
   Asn Glu Glu Gln Gly Met Val Thr Gln Trp Gln Ser Leu Phe Tyr Lys
                   565                 570                 575
   His Arg Tyr Ser His Thr His Gln Leu Asn Pro Asp Phe Val Lys Leu
               580                 585                 590
   Ala Glu Ala Met Gly Phe Lys Ala Leu Arg Leu Asn Lys Gln Glu Asp
   595 600 605
   Val Asp Ala Lys Leu Lys Glu Phe Val Asp Cys Glu Gly Pro Val Leu
   610 615 620
   Leu Glu Val Glu Val Glu Lys Lys Val Pro Val Leu Pro Met Val Pro
   625 630 635 640
   Ala Gly Lys Gly Leu Asp Glu Phe Ile Lys Phe Asp Pro Glu Thr Glu
   645 650 655
   Arg Glu Gln Lys Glu Leu Arg His Lys Arg Thr Gln Gly Lys His
   660 665 670
<210> 31
   <211> 700
   <212> PRT
   <213> Zygosaccharomyces rouxii
<400> 31
   Met Ile Arg Gln Ala Arg Gln Met Gly Ala Thr Arg Cys Ala Val Arg
   1 5 10 15
   Tyr Ala Val Arg Asn Ile Ser Ser Asn Ser Ala Arg Ser Ser Arg Asn
   20 25 30
   Leu Ala Tyr Ile Asn Thr Val Arg Tyr His Ser Thr Thr Arg Lys Gln
   35 40 45
   Gln Tyr Ser Ala Ala Thr Ala Thr Gly Glu Ser Thr Ala Thr Ser Asn
   50 55 60
   Ala Thr Ala Arg Arg Pro Ala Pro Ala Pro Ser Phe Asn Val Asp Leu
   65 70 75 80
   Thr Gly Thr Pro Asp Lys Leu His Lys Lys Leu Lys Ser Gln Gln Glu
   85 90 95
   Met Asp Ser Ser Phe Ile Gly Leu Thr Gly Gly Gln Ile Phe His Glu
   100 105 110
   Met Met Arg Arg His Asp Val Asp Thr Val Phe Gly Tyr Pro Gly Gly
   115 120 125
   Ala Ile Leu Pro Val Tyr Asp Ala Ile His Glu Ser Asp Ala Phe Asn
   130 135 140
   Phe Ile Leu Pro Arg His Glu Gln Gly Ala Gly His Met Ala Glu Gly
   145                 150                 155                 160
   Tyr Ala Arg Ala Thr Gly Arg Pro Gly Val Val Leu Val Thr Ser Gly
                   165                 170                 175
   Pro Gly Ala Thr Asn Val Val Thr Pro Met Thr Asp Ala Leu Ala Asp
               180                 185                 190
   Gly Val Pro Leu Ile Val Phe Thr Gly Gln Val Ala Thr Ser Ala Ile
           195                 200                 205
   Gly Thr Asp Ala Phe Gln Glu Ala Asp Val Val Gly Ile Ser Arg Ser
       210                 215                 220
   Cys Thr Lys Trp Asn Val Met Val Lys Asn Val Glu Glu Leu Pro Lys
   225                 230                 235                 240
   Arg Ile Asn Glu Ala Phe Glu Ile Ala Met Ser Gly Arg Pro Gly Pro
                   245                 250                 255
   Val Leu Val Asp Leu Pro Lys Asp Val Thr Ala Ala Val Leu Arg Asn
               260                 265                 270
   Pro Ile Pro Thr Lys Ser Thr Leu Pro Ser Asp Ser Leu Ala Gln Leu
           275                 280                 285
   Thr Lys Arg Ala Gln Asp Glu Phe Thr Ser Asn Asn Val Lys Arg Ala
       290                 295                 300
   Ala Asp Leu Val Asn Val Ala Lys Lys Pro Ile Leu Tyr Val Gly Ala
   305                 310                 315                 320
   Gly Ile Phe Asn Asn Pro Asp Gly Pro Arg Leu Leu Lys Glu Leu Ser
                   325                 330                 335
   Glu Arg Ala Gln Ile Pro Val Thr Thr Thr Leu Gln Gly Leu Gly Ala
               340                 345                 350
   Phe Asp Gln Glu Asp Thr Lys Ser Leu Asp Met Leu Gly Met His Gly
           355                 360                 365
   Cys Ala Thr Ala Asn Leu Ala Ile Gln Asn Ala Asp Leu Ile Leu Ala
       370                 375                 380
   Val Gly Ala Arg Phe Asp Asp Arg Val Thr Cys Asn Ile Ser Lys Phe
   385 390 395 400
   Ala Pro Glu Ala Arg Arg Ala Ala Leu Glu Gly Arg Gly Gly Ile Val
   405 410 415
   His Phe Glu Met Thr Pro Lys Asn Ile Ser Lys Val Val Glu Thr Gln
   420 425 430
   Val Ala Val His Gly Asp Ala Ala Thr Asn Ile Gln Lys Leu Ile Pro
   435 440 445
   Leu Val Phe Gly Val Lys Glu Arg Gln Glu Trp Phe Asn Gln Ile Ser
   450 455 460
   Gln Trp Lys Lys Glu Tyr Pro Leu Asp Tyr Met His Glu Thr Pro Gly
   465 470 475 480
   Ser Lys Ile Lys Pro Gln Thr Val Ile Ser Lys Leu Ser Asp Ile Ala
   485 490 495
   Asn Ala Thr Gly Lys Glu Val Ile Val Thr Thr Gly Val Gly Gln His
   500 505 510
   Gln Met Trp Ala Ala Gln His Trp Thr Trp Lys Thr Pro Arg Ser Phe
   515 520 525
   Ile Thr Ser Gly Gly Leu Gly Thr Met Gly Tyr Gly Leu Pro Ala Ala
   530 535 540
   Ile Gly Ala Gln Ile Ala Lys Pro Asn Ala Leu Val Phe Asp Ile Asp
   545 550 555 560
   Gly Asp Ala Ser Phe Asn Met Thr Leu Thr Glu Leu Ser Ser Ala Val
   565 570 575
   Gln Ala Lys Thr Pro Ile Lys Ile Leu Val Leu Asn Asn Glu Glu Gln
   580 585 590
   Gly Met Val Thr Gln Trp Gln Ser Leu Phe Tyr Glu His Arg Tyr Ser
   595 600 605
   His Thr His Gln Leu Asn Pro Asp Phe Val Lys Leu Ser Glu Ala Met
   610 615 620
   Gly Leu Lys Gly Met Arg Leu Thr Asp Gln Ser Lys Thr Asp Glu Val
   625 630 635 640
   Leu Lys Glu Phe Val Glu Tyr Asn Asp Gly Pro Val Leu Leu Glu Val
                   645                 650                 655
   Glu Val Ala Pro Lys Val Pro Val Leu Pro Met Val Pro Gly Gly Lys
               660                 665                 670
   Gly Leu Glu Glu Phe Ile Asn Tyr Asp Pro Glu Met Glu Glu Glu Gln
           675                 680                 685
   Asn Arg Leu Arg Tyr Glu Arg Thr Ser Gly Lys Tyr
       690                 695                 700
<210> 32
   <211> 685
   <212> PRT
   <213> Zygosaccharomyces bailii
<400> 32
   Met Ile Arg Gln Ala Thr Val Lys Gln Met Gly Ala Thr Arg Cys Ala
   1               5                   10                  15
   Val Arg Ser Ala Val Arg Thr Thr Thr Arg Asn Phe Ala Cys Tyr Thr
               20                  25                  30
   Val Arg Tyr His Ser Thr Thr Ala Pro Lys Gln Tyr Ser Ala Ala Thr
           35                  40                  45
   Ala Gly Ala Lys Arg Pro Glu Pro Ala Pro Ser Phe Asn Met Glu Pro
       50                  55                  60
   Thr Gly Thr Pro Ser Lys Leu His Gln Lys Leu Lys Asn Asn Glu Gln
   65                  70                  75                  80
   Gln Met Asp Ser Ser Phe Ile Gly Leu Thr Gly Gly Gln Ile Phe His
                   85                  90                  95
   Glu Met Met Arg Arg His Asp Val Asp Thr Val Phe Gly Tyr Pro Gly
               100                 105                 110
   Gly Ala Ile Leu Pro Val Tyr Asp Ala Ile His Asp Ser Glu Ala Phe
           115                 120                 125
   Asn Phe Val Leu Pro Arg His Glu Gln Gly Ala Gly His Met Ala Glu
       130                 135                 140
   Gly Tyr Ala Arg Ala Thr Gly Lys Pro Gly Ile Val Leu Val Thr Ser
   145                 150                 155                 160
   Gly Pro Gly Ala Thr Asn Val Val Thr Pro Met Thr Asp Ala Leu Ala
                   165                 170                 175
   Asp Gly Val Pro Leu Ile Val Phe Thr Gly Gln Val Pro Thr Ser Ala
               180                 185                 190
   Ile Gly Thr Asp Ala Phe Gln Glu Ala Asp Val Val Gly Ile Ser Arg
           195                 200                 205
   Ser Cys Thr Lys Trp Asn Val Met Val Lys Ser Val Asp Glu Leu Pro
       210                 215                 220
   Arg Arg Ile Asn Glu Ala Phe Glu Ile Ala Met Ser Gly Arg Pro Gly
   225                 230                 235                 240
   Pro Val Leu Val Asp Leu Pro Lys Asp Val Thr Ala Ala Val Leu Arg
                   245                 250                 255
   Asn Pro Ile Pro Thr Lys Ser Thr Leu Pro Ser Asp Ser Leu Ala Gln
               260                 265                 270
   Leu Thr Lys Gly Ala His Asp Glu Phe Thr Ser Asn Asn Val Lys Arg
           275                 280                 285
   Ala Ala Asp Leu Ile Asn Val Ala Lys Lys Pro Ile Leu Tyr Val Gly
       290                 295                 300
   Gly Gly Ile Phe Asn Asn Pro Asp Gly Pro Arg Leu Ile Arg Glu Leu
   305                 310                 315                 320
   Ser Glu Arg Ala Gln Ile Pro Val Thr Thr Thr Leu Gln Gly Leu Gly
                   325                 330                 335
   Ala Phe Asp Gln Glu Asp Val Lys Ser Leu Asp Met Leu Gly Met His
               340                 345                 350
   Gly Cys Ala Thr Ala Asn Leu Ala Val Gln Asn Ala Asp Leu Ile Ile
           355                 360                 365
   Ala Val Gly Ala Arg Phe Asp Asp Arg Val Thr Cys Asn Ile Ser Lys
       370                 375                 380
   Phe Ala Pro Glu Ala Arg Arg Ala Ala Leu Glu Asn Arg Gly Gly Ile
   385                 390                 395                 400
   Ile His Phe Glu Met Ser Pro Lys Asn Ile Ser Lys Val Val Glu Thr
                   405                 410                 415
   Gln Val Ala Val His Gly Asp Ala Ala Thr Asn Leu Glu Lys Leu Ile
               420                 425                 430
   Pro Leu Val Phe Gly Val Lys Glu Arg Lys Gln Trp Phe Asp Gln Ile
           435                 440                 445
   Ala Arg Trp Lys Glu Glu Tyr Pro Leu Asp Tyr Met His Glu Thr Pro
       450                 455                 460
   Gly Ser Lys Ile Lys Pro Gln Thr Val Ile Ala Lys Leu Ser Lys Ile
   465                 470                 475                 480
   Ala Asn Ser Leu Gly Lys Pro Val Ile Val Thr Thr Gly Val Gly Gln
                   485                 490                 495
   His Gln Met Trp Ala Ala Gln His Trp Thr Trp Lys Ser Pro Arg Thr
               500                 505                 510
   Phe Ile Thr Ser Gly Gly Leu Gly Thr Met Gly Tyr Gly Leu Pro Ala
           515                 520                 525
   Ala Ile Gly Ala Gln Ile Ala Arg Pro Asp Ala Ile Val Phe Asp Ile
       530                 535                 540
   Asp Gly Asp Ala Ser Phe Asn Met Ser Leu Thr Glu Met Ser Ser Ala
   545                 550                 555                 560
   Val Gln Ala Lys Ala Pro Ile Lys Ile Ile Ile Leu Asn Asn Glu Glu
                   565                 570                 575
   Gln Gly Met Val Thr Gln Trp Gln Ser Leu Phe Tyr Glu His Arg Tyr
               580                 585                 590
   Ser His Thr His Gln Leu Asn Pro Asp Phe Val Lys Leu Ala Glu Ala
           595                 600                 605
   Met Gly Met Lys Gly Met Arg Leu Ser Asp Gln Ser Lys Thr Asp Glu
       610                 615                 620
   Val Leu Lys Glu Phe Val Asp Tyr Ser Glu Gly Pro Val Leu Leu Glu
   625                 630                 635                 640
   Val Glu Val Ala Thr Lys Val Pro Val Leu Pro Met Val Pro Gly Gly
                   645                 650                 655
   Lys Gly Leu Glu Glu Phe Ile Asn Tyr Asp Pro Glu Met Glu Lys Glu
               660                 665                 670
   Gln Asn Arg Leu Arg Tyr Glu Arg Thr Lys Gly Lys Tyr
   675 680 685
<210> 33
   <211> 691
   <212> PRT
   <213> Kluyveromyces lactis
<400> 33
   Met Leu Ser Gln Arg Phe Val Thr Arg Ala Leu Ala Arg Arg Ala Phe
   1 5 10 15
   Gly Lys Thr Cys Leu Lys Leu Gly Ser Ala Ser Ala Ser Ala Ser Asn
   20 25 30
   Ala Ala Ser Ala Ala Ala Gly Glu Ala Ser Ser Arg Pro Gln Pro Ala
   35 40 45
   Pro Ser Phe Asn Ala Asp Ile Ala Gly Gly Ser Ser Gly Gly Ala Arg
   50 55 60
   Arg Asn Gln Ile Pro Leu Ser Lys Arg Ser Arg Ile Ala Asp Lys Asn
   65 70 75 80
   Thr Gly Val Asn Ile Gln Asn Gln Met Asp Asp Ser Phe Ile Gly Met
   85 90 95
   Thr Gly Gly Glu Ile Phe His Glu Met Met Gln Arg His Asn Val Asp
   100 105 110
   Thr Val Phe Gly Tyr Pro Gly Gly Ala Ile Leu Pro Val Tyr Asp Ala
   115 120 125
   Ile Tyr Asn Ser Asp Lys Phe Thr Phe Val Leu Pro Lys His Glu Gln
   130 135 140
   Gly Ala Gly His Met Ala Glu Gly Tyr Ala Arg Ala Ser Gly Lys Pro
   145 150 155 160
   Gly Val Val Leu Val Thr Ser Gly Pro Gly Ala Thr Asn Val Val Thr
   165 170 175
   Pro Met Ala Asp Ala Leu Ala Asp Gly Val Pro Leu Val Val Phe Thr
   180 185 190
   Gly Gln Val Ala Thr Ser Ala Ile Gly Thr Asp Ala Phe Gln Glu Ala
   195 200 205
   Asp Val Val Gly Ile Ser Arg Ser Cys Thr Lys Trp Asn Val Met Val
       210                 215                 220
   Lys Thr Ile Glu Glu Leu Pro Arg Arg Ile Asn Glu Ala Phe Glu Ile
   225                 230                 235                 240
   Ala Thr Ser Gly Arg Pro Gly Pro Val Leu Val Asp Leu Pro Lys Asp
                   245                 250                 255
   Val Thr Ala Ala Val Leu Arg Asn Pro Ile Pro Met Lys Ser Ile Leu
               260                 265                 270
   Pro Ser Asp Thr Leu Asn Gln Leu Thr Gln Arg Val Val Asp Glu Phe
           275                 280                 285
   Thr Leu Glu Asn Ile Ser Arg Ala Ala Asp Leu Ile Asn Arg Ala Lys
       290                 295                 300
   Lys Pro Leu Leu Tyr Val Gly Ala Gly Ile Leu Gly Ser Glu Met Gly
   305                 310                 315                 320
   Pro Gln Tyr Leu Lys Gln Leu Ser Asp Arg Ala Gln Ile Pro Val Thr
                   325                 330                 335
   Thr Thr Leu Gln Gly Leu Gly Ala Phe Asn Gln Glu Asp His Lys Ser
               340                 345                 350
   Leu Asp Met Val Gly Met His Gly Asn Ala Ala Ala Asn Leu Ala Ile
           355                 360                 365
   Gln Asn Ala Asp Leu Ile Ile Ala Leu Gly Ala Arg Phe Asp Asp Arg
       370                 375                 380
   Val Thr Leu Asn Ile Ser Lys Phe Ala Pro Glu Ala Arg Lys Ala Ala
   385                 390                 395                 400
   Leu Glu Lys Arg Gly Gly Ile Val His Phe Glu Ile Ser Pro Lys Asn
                   405                 410                 415
   Ile Asn Lys Val Val Glu Ala Glu Val Ala Val Glu Gly Asp Val Thr
               420                 425                 430
   Arg Asn Leu Glu Lys Leu Leu Pro Leu Val Glu Ser Val Pro Glu Arg
           435                 440                 445
   Pro Glu Trp Ser Ala Gln Val Ala Lys Trp Lys Gln Glu Tyr Pro Tyr
   450 455 460
   Ala Tyr Gln Lys Glu Thr Pro Gly Ser Lys Ile Lys Pro Gln Thr Val
   465 470 475 480
   Ile Ser Lys Leu Ser Asp Leu Val Leu Lys Ser Gly Arg Pro Ala Ile
   485 490 495
   Val Thr Thr Gly Val Gly Gln His Gln Met Trp Ala Ala Gln His Trp
   500 505 510
   Thr Trp Lys Ser Pro Arg Ser Phe Ile Thr Ser Gly Gly Leu Gly Thr
   515 520 525
   Met Gly Tyr Gly Leu Pro Ala Ala Ile Gly Ala Gln Val Ala Arg Pro
   530 535 540
   Asp Ala Ile Val Ile Asp Ile Asp Gly Asp Ala Ser Phe Asn Met Thr
   545 550 555 560
   Leu Met Glu Leu Ser Ser Ala Val Gln Ala Arg Thr Pro Val Lys Val
   565 570 575
   Leu Leu Leu Asn Asn Glu Glu Gln Gly Met Val Thr Gln Trp Gln Ser
   580 585 590
   Leu Phe Tyr Glu His Arg Tyr Ser His Thr His Gln Leu Asn Pro Asp
   595 600 605
   Phe Val Lys Leu Ala Glu Ala Met Gly Leu Lys Ala Leu Arg Leu Ser
   610 615 620
   Lys Gln Glu Asp Thr Asp Ala Ile Leu Lys Glu Phe Val Asp Tyr Glu
   625 630 635 640
   Gly Pro Ile Leu Leu Glu Val Gln Ile Glu Lys Lys Val Pro Val Leu
   645 650 655
   Pro Met Val Pro Thr Gly Lys Gly Leu His Glu Phe Ile Asn Phe Asp
   660 665 670
   Pro Glu Val Glu Lys Glu Gln Asn Glu Leu Arg His Lys Arg Thr Ser
   675 680 685
   Gly Lys Tyr
   690
<210> 34
   <211> 700
   <212> PRT
   <213> Kluyveromyces marxianus
<400> 34
   Met Leu Ala Gly Arg Ile Ala Ser Gln Arg Ser Ala Arg Ile His Ser
   1               5                   10                  15
   Ile His Lys Leu Tyr Lys His Ser Arg Cys Ile Ala Met Ile Gly Gly
               20                  25                  30
   Ala Arg Ala Pro Pro Ile His Met Val Pro Thr Gly Thr Arg Ser Ala
           35                  40                  45
   Ala Ser Ala Ala Ala Val Ser Ser Ala Glu Thr Ser Asn Glu Gln Pro
       50                  55                  60
   Ala Arg Pro Gln Pro Ala Pro Ser Phe Asn Ile Pro Ser Leu Lys Asn
   65                  70                  75                  80
   Arg Thr Thr Thr Asn Ser Asn Ser Lys Asp Lys His Gly Leu Ala Gln
                   85                  90                  95
   Met Asp Asp Ser Phe Ile Gly Met Thr Gly Gly Glu Ile Phe His Glu
               100                 105                 110
   Met Met Gln Arg His Asn Val Asp Thr Val Phe Gly Tyr Pro Gly Gly
           115                 120                 125
   Ala Ile Leu Pro Val Tyr Asp Ala Ile Tyr Asn Ser Asp Lys Phe Lys
       130                 135                 140
   Phe Val Leu Pro Arg His Glu Gln Gly Ala Gly His Met Ala Glu Gly
   145                 150                 155                 160
   Tyr Ala Arg Ala Ser Gly Lys Pro Gly Val Val Leu Val Thr Ser Gly
                   165                 170                 175
   Pro Gly Ala Thr Asn Val Val Thr Pro Met Ala Asp Ala Leu Ala Asp
               180                 185                 190
   Gly Val Pro Leu Val Val Phe Thr Gly Gln Val Ala Thr Ser Ala Ile
           195                 200                 205
   Gly Thr Asp Ala Phe Gln Glu Ala Asp Val Val Gly Ile Ser Arg Ser
       210                 215                 220
   Cys Thr Lys Trp Asn Val Met Val Arg Ser Ile Glu Glu Leu Pro Arg
   225                 230                 235                 240
   Arg Ile Asn Glu Ala Phe Glu Ile Ala Thr Ser Gly Arg Pro Gly Pro
                   245                 250                 255
   Val Leu Val Asp Leu Pro Lys Asp Val Thr Ala Ala Val Leu Arg Asn
               260                 265                 270
   Pro Ile Pro Met Lys Ser Ile Leu Pro Ser Asp Thr Leu Asn Gln Leu
           275                 280                 285
   Thr Gln Arg Val Val Asp Glu Tyr Thr Leu Gln Asn Ile Gly Arg Ala
       290                 295                 300
   Ala Glu Leu Ile Asn Lys Ala Lys Lys Pro Leu Leu Tyr Val Gly Ala
   305                 310                 315                 320
   Gly Ile Leu Gly His Ser Lys Gly Pro Gln Phe Leu Lys Gln Leu Ser
                   325                 330                 335
   Asp Arg Ala Gln Ile Pro Val Thr Thr Thr Leu Gln Gly Leu Gly Ala
               340                 345                 350
   Phe Asp Gln Glu Asp Pro Lys Ser Leu Asp Met Val Gly Met His Gly
           355                 360                 365
   Asn Ala Ala Ala Asn Leu Ala Ile Gln Asn Ala Asp Leu Ile Ile Ala
       370                 375                 380
   Leu Gly Ala Arg Phe Asp Asp Arg Val Thr Leu Asn Ile Ser Lys Phe
   385                 390                 395                 400
   Ala Pro Glu Ala Arg Lys Ala Ala Leu Glu Lys Arg Gly Gly Ile Val
                   405                 410                 415
   His Phe Glu Ile Ser Pro Lys Asn Ile Asn Lys Val Val Glu Ala Glu
               420                 425                 430
   Val Ala Val Glu Gly Asp Val Thr His Asn Leu Glu Lys Leu Leu Pro
           435                 440                 445
   Leu Ile Glu Thr Val Pro Glu Arg Pro Glu Trp Ser Ala Gln Val Gln
       450                 455                 460
   Glu Trp Lys Gln Lys Tyr Pro Tyr Ser Tyr Gln Arg Glu Thr Pro Gly
   465                 470                 475                 480
   Ser Lys Ile Lys Pro Gln Thr Val Ile Ser Lys Leu Ser Lys Leu Val
                   485                 490                 495
   Asn Ser Thr Gly Lys Pro Ala Ile Val Thr Thr Gly Val Gly Gln His
               500                 505                 510
   Gln Met Trp Ala Ala Gln Gln Trp Thr Trp Lys Ser Pro Arg Thr Phe
           515                 520                 525
   Ile Thr Ser Gly Gly Leu Gly Thr Met Gly Tyr Gly Leu Pro Ala Ala
       530                 535                 540
   Ile Gly Ala Gln Val Ala Arg Pro Asp Ala Ile Val Ile Asp Ile Asp
   545                 550                 555                 560
   Gly Asp Ala Ser Phe Asn Met Thr Leu Met Glu Leu Ser Ser Ala Val
                   565                 570                 575
   Gln Ala Arg Thr Pro Ile Lys Ile Leu Leu Leu Asn Asn Glu Glu Gln
               580                 585                 590
   Gly Met Val Thr Gln Trp Gln Ser Leu Phe Tyr Glu His Arg Tyr Ser
           595                 600                 605
   His Thr His Gln Leu Asn Pro Asp Phe Val Lys Leu Ala Glu Ala Met
       610                 615                 620
   Gly Leu Lys Ala Leu Arg Leu Ser Asp Gln Lys Asp Thr Asp Ala Val
   625                 630                 635                 640
   Leu Lys Gln Phe Ile Asp Tyr Asn Asp Gly Pro Ile Leu Leu Glu Val
                   645                 650                 655
   Gln Val Glu Lys Lys Val Pro Val Leu Pro Met Val Pro Thr Gly Lys
               660                 665                 670
   Gly Leu His Glu Phe Ile Asn Phe Asp Pro Glu Glu Glu Lys Lys Gln
           675                 680                 685
   Asn Glu Leu Arg His Lys Arg Thr Asn Gly Lys His
       690                 695                 700
<210> 35
   <211> 678
   <212> PRT
   <213> Lachancea thermotolerans
<400> 35
   Met Met Arg Gln Ser Ala Gly Lys Met Met Leu Arg Arg Gly Ile Ser
   1               5                   10                  15
   Ala Ala Leu Ala Arg Asn Ser Ala Arg Ile Gly Ser Leu Arg Leu Tyr
               20                  25                  30
   Ser Ala Ser Ala Thr Ser Thr Gln Thr Ala Asp Asp Gln Arg Pro Arg
           35                  40                  45
   Pro Ala Pro Ser Phe Asp Met Glu Pro Ser Arg Ser Val Lys Ala Ser
       50                  55                  60
   Lys Leu Arg Lys Ser Glu Met Pro Leu Glu Ala Met Asp Asp Ser Phe
   65                  70                  75                  80
   Ile Gly Leu Ser Gly Gly Gln Ile Phe His Glu Met Met Arg Arg His
                   85                  90                  95
   Lys Val Asn Thr Val Phe Gly Tyr Pro Gly Gly Ala Ile Leu Ala Val
               100                 105                 110
   Tyr Asp Ala Ile Tyr Asn Ser Glu His Phe Asn Phe Val Leu Pro Lys
           115                 120                 125
   His Glu Gln Gly Ala Gly His Met Ala Glu Gly Tyr Ala Arg Val Ser
       130                 135                 140
   Gly Lys Pro Gly Val Val Leu Val Thr Ser Gly Pro Gly Ala Thr Asn
   145                 150                 155                 160
   Val Val Thr Pro Met Ala Asp Ala Leu Ala Asp Gly Val Pro Met Val
                   165                 170                 175
   Val Phe Thr Gly Gln Val Ala Thr Ser Ser Ile Gly Thr Asp Ala Phe
               180                 185                 190
   Gln Glu Ala Asp Val Ile Gly Ile Ser Arg Ser Cys Thr Lys Trp Asn
           195                 200                 205
   Val Met Val Lys Asn Val Glu Glu Leu Pro Arg Arg Ile Asn Glu Ala
       210                 215                 220
   Phe Glu Ile Ala Val Ser Gly Arg Pro Gly Pro Val Leu Val Asp Leu
   225                 230                 235                 240
   Pro Lys Asp Val Thr Ser Ala Ile Leu Arg Thr Pro Ile Pro Val Lys
                   245                 250                 255
   Ser Thr Leu Pro Ser Asn Ala Leu Ser Lys Ile Thr Lys Ser Ala Ala
               260                 265                 270
   Lys Glu Phe Thr Leu Ala Asn Leu Gln Arg Ala Ala Asp Leu Ile Asn
           275                 280                 285
   Ile Ala Lys Lys Pro Leu Ile Tyr Val Gly Gly Gly Ile Phe Gly Lys
       290                 295                 300
   Glu Gln Gly Pro Lys Leu Leu Arg Glu Leu Ala Glu Arg Ala Gln Ile
   305                 310                 315                 320
   Pro Val Ser Thr Thr Ile Gln Gly Leu Gly Ala Phe Asp Gln Glu Asp
                   325                 330                 335
   Pro Lys Ser Leu Asp Met Leu Gly Met His Gly Ser Ala Phe Ala Asn
               340                 345                 350
   Leu Ser Val Gln Asn Ala Asp Leu Ile Ile Ala Leu Gly Ala Arg Phe
           355                 360                 365
   Asp Asp Arg Val Thr Cys Asn Val Ala Lys Phe Ala Pro Glu Ala Arg
       370                 375                 380
   Lys Ala Ala Leu Glu Asn Arg Gly Gly Ile Ile His Phe Glu Ile Ser
   385                 390                 395                 400
   Pro Lys Asn Ile Asn Lys Val Val Glu Ala Gln Val Ala Val Glu Gly
                   405                 410                 415
   Asp Val Thr Asp Asn Leu Glu Arg Leu Ile Pro Leu Val Asn Asn Val
               420                 425                 430
   Lys Glu Arg Lys Glu Trp Phe Ala Gln Ile Asp Ala Trp Lys Lys Glu
           435                 440                 445
   Tyr Pro Tyr Asp Tyr Gln Arg Glu Thr Lys Asp Ser Lys Ile Lys Pro
       450                 455                 460
   Gln Thr Leu Val Ala Arg Leu Ser Glu Val Ala Ser Ser Thr Gly Arg
   465                 470                 475                 480
   Asp Val Ile Val Thr Thr Gly Val Gly Gln His Gln Met Trp Ala Ala
                   485                 490                 495
   Gln His Trp Thr Trp Arg His Pro Arg Thr Phe Ile Thr Ser Gly Gly
   500 505 510
   Leu Gly Thr Met Gly Tyr Gly Leu Pro Ser Ala Ile Gly Ala Gln Ile
   515 520 525
   Ala Arg Pro Asp Ala Leu Val Ile Asp Ile Asp Gly Asp Ala Ser Phe
   530 535 540
   Asn Met Thr Leu Met Glu Leu Ser Ser Ala Val Gln Ala Gly Ala Pro
   545 550 555 560
   Val Lys Ile Val Val Leu Asn Asn Glu Glu Gln Gly Met Val Thr Gln
   565 570 575
   Trp Gln Ser Leu Phe Tyr Glu His Arg Tyr Ser His Thr His Gln Leu
   580 585 590
   Asn Pro Asp Phe Val Lys Leu Ala Glu Ala Met Gly Leu Lys Gly Met
   595 600 605
   Arg Leu Ser Lys Gln Glu Asp Met Asp Ser Val Leu Lys Glu Phe Val
   610 615 620
   Asn Thr Glu Gly Pro Val Leu Leu Glu Val Ile Val Glu Lys Lys Val
   625 630 635 640
   Pro Val Leu Pro Met Val Pro Ala Gly Lys Gly Leu His Glu Phe Ile
   645 650 655
   Asn Phe Asp Ala Glu Val Glu Lys Glu Gln Asn Glu Leu Arg His Lys
   660 665 670
   Arg Thr Gly Gly Lys His
   675
<210> 36
   <211> 688
   <212> PRT
   <213> Tetrapisispora phaffia
<400> 36
   Met Leu Arg Ser Thr Gly Ser Arg Thr Ser Leu Asn Arg Leu Ala Lys
   1               5                   10                  15
   Ile Ser Lys Ser Val Asp Ser Gln Trp Phe Val Thr Ala Thr Leu Leu
               20                  25                  30
   Lys Arg Asp Phe Thr Ser Ser Gln Leu Ser Leu Lys Ser Ser Val Tyr
               35                  40                  45
   Ser Arg Pro Ser Pro Ala Pro Ser Phe Asn Ala Ser Pro Thr Leu Ala
       50                  55                  60
   Asp Ser Thr Asn Lys Ile Ser Gln Ser Lys Leu Lys Glu Leu Lys Lys
   65                  70                  75                  80
   Asn Pro Glu Met Asp Ser Ser Leu Ile Gly Leu Thr Gly Gly Glu Ile
                   85                  90                  95
   Phe His Glu Met Met Lys Arg His Asn Val Asp Thr Val Phe Gly Tyr
               100                 105                 110
   Pro Gly Gly Ala Ile Leu Pro Val Tyr Asp Ala Ile Tyr Asn Ser Lys
           115                 120                 125
   Asp Phe Asn Phe Val Leu Pro Arg His Glu Gln Gly Ala Gly His Met
       130                 135                 140
   Ala Glu Gly Tyr Ala Arg Ala Ser Gly Lys Cys Gly Val Val Leu Val
   145                 150                 155                 160
   Thr Ser Gly Pro Gly Ala Thr Asn Val Ile Thr Pro Met Ala Asp Ala
                   165                 170                 175
   Leu Ala Asp Gly Val Pro Met Val Val Phe Thr Gly Gln Val Pro Thr
               180                 185                 190
   Ala Ala Ile Gly Thr Asp Ala Phe Gln Glu Ala Asp Val Ile Gly Ile
           195                 200                 205
   Ser Arg Ser Cys Thr Lys Trp Asn Val Met Val Lys Arg Val Glu Asp
       210                 215                 220
   Leu Pro Lys Cys Ile Asn Glu Ala Phe Glu Ile Ala Thr Ser Gly Arg
   225                 230                 235                 240
   Pro Gly Pro Val Leu Val Asp Leu Pro Lys Asp Val Gln Ala Ser Ile
                   245                 250                 255
   Leu Arg Ala Pro Ile Pro Ile Lys Thr Thr Ile Pro Ser Asn Ala Leu
               260                 265                 270
   Lys Gln Leu Thr Arg Ser Ala Tyr Thr Gln Phe Ser Glu Ala Gly Ile
           275                 280                 285
   Lys Arg Ala Ala Glu Leu Ile Asn Ile Ala Lys Lys Pro Ile Leu Tyr
       290                 295                 300
   Val Gly Gly Gly Ile Leu Asn Asn Pro Glu Gly Pro Arg Leu Ile Lys
   305                 310                 315                 320
   Glu Leu Ser Asp Arg Ala Gln Ile Pro Val Thr Thr Thr Leu Gln Ala
                   325                 330                 335
   Leu Gly Ala Phe Asp Gln Asp Asp Pro Lys Ser Leu Asp Met Leu Gly
               340                 345                 350
   Met His Gly Tyr Ala Pro Ala Asn Leu Ala Ile Gln Asn Ala Asp Leu
           355                 360                 365
   Ile Ile Ala Val Gly Ala Arg Phe Asp Asp Arg Val Thr Leu Asn Ile
       370                 375                 380
   Ser Lys Phe Ala Pro Glu Ala Arg Arg Ala Ala Leu Glu Asn Arg Gly
   385                 390                 395                 400
   Gly Ile Ile His Phe Asp Ile Ser Ala Lys Asn Ile Asn Lys Val Val
                   405                 410                 415
   Glu Thr Gln Val Pro Ile Glu Gly Asp Ala Ala Thr Asn Leu Gln Gln
               420                 425                 430
   Met Ile Pro Ser Val Phe Ser Val Thr Glu Arg Glu Arg Lys Glu Trp
           435                 440                 445
   Leu Asp Thr Ile Asn Asn Trp Lys Ala Lys Tyr Pro Tyr Ala Tyr Gln
       450                 455                 460
   Lys Glu Thr Pro Gly Ser Lys Ile Lys Pro Gln Thr Val Ile Ala Lys
   465                 470                 475                 480
   Leu Ser Glu Ile Ala Asn Ser Ser Gly Lys Pro Ile Val Val Thr Thr
                   485                 490                 495
   Gly Val Gly Gln His Gln Met Trp Ala Ala Gln His Trp Thr Trp Lys
               500                 505                 510
   Thr Pro Arg Thr Phe Ile Ser Ser Gly Gly Leu Gly Thr Met Gly Tyr
           515                 520                 525
   Gly Leu Pro Ala Ala Ile Gly Ala Gln Val Ala Arg Pro Asp Ala Val
       530                 535                 540
   Val Ile Asp Ile Asp Gly Asp Ala Ser Phe Asn Met Ser Leu Thr Glu
   545 550 555 560
   Leu Ser Ser Ala Val Gln Ala Cys Thr Pro Ile Lys Ile Leu Leu Leu
   565 570 575
   Asn Asn Glu Glu Gln Gly Met Val Thr Gln Trp Gln Ser Leu Phe Tyr
   580 585 590
   Lys His Arg Tyr Ser His Thr His Gln Leu Asn Pro Asp Phe Val Lys
   595 600 605
   Ile Ala Glu Ala Met Gly Met Lys Gly Met Arg Leu Ala Glu Gln Ser
   610 615 620
   Lys Met Glu Asp Thr Leu His Glu Phe Phe Asn Phe Asp Gly Pro Val
   625 630 635 640
   Leu Leu Glu Val Val Val Glu Lys Lys Val Pro Val Leu Pro Met Val
   645 650 655
   Pro Ala Gly Lys Gly Leu Asp Glu Phe Ile Cys Phe Asp Pro Glu Val
   660 665 670
   Glu Lys Glu Gln Asn Glu Leu Arg Arg Lys Arg Thr Asn Gly Lys His
   675 680 685
<210> 37
   <211> 710
   <212> PRT
   <213> Tetrapisispora blattae
<400> 37
   Met Ser Leu Ser Ala Ile Ser Arg Thr Arg Ser Val Leu Ser Lys Trp
   1 5 10 15
   Ser Phe Gln Ser Thr Thr Leu His Asn Arg Pro Cys Ala Thr Arg Val
   20 25 30
   Phe Thr Arg Thr Leu Ala Pro Gln Arg Lys His Leu Ser Thr Ser Phe
   35 40 45
   Ile Thr Gln Ala Ser Ser Pro Lys His Lys Arg Ser Glu Lys Val Lys
   50 55 60
   Pro Asn Asp Leu Asp Leu Glu Phe Glu Gln Asn Lys Arg Pro Glu Pro
   65 70 75 80
   Ser Pro Ser Phe Asp Val Asp Pro Ala Pro Lys Leu Asp Gly Thr Ala
   85 90 95
   Ala Asn Ala Lys Asn Ala Asp Ser Pro Asp Leu Asp His Thr Met Ile
   100 105 110
   Gly Met Thr Gly Gly Glu Ile Phe Asn Glu Met Met Arg Arg Lys Asn
   115 120 125
   Val Asp Thr Val Phe Gly Tyr Pro Gly Gly Ala Ile Leu Pro Val Tyr
   130 135 140
   Asp Ala Ile Tyr Asn Ser Glu Asn Phe Lys Phe Val Leu Pro Arg His
   145 150 155 160
   Glu Gln Gly Ala Gly His Met Ala Glu Gly Tyr Ala Arg Ala Ser Gly
   165 170 175
   Lys Pro Gly Val Val Leu Val Thr Ser Gly Pro Gly Ala Thr Asn Val
   180 185 190
   Val Thr Pro Met Ala Asp Ala Leu Ala Asp Gly Val Pro Met Val Val
   195 200 205
   Phe Thr Gly Gln Val Pro Thr Ser Ala Ile Gly Thr Asp Ala Phe Gln
   210 215 220
   Glu Ala Asp Val Ile Gly Ile Ser Arg Ser Cys Thr Lys Trp Asn Val
   225 230 235 240
   Met Val Lys Ser Val Asp Glu Leu Pro Lys Arg Ile Asn Glu Ala Phe
   245 250 255
   Glu Ile Ala Met Ser Gly Arg Pro Gly Pro Val Leu Ile Asp Leu Pro
   260 265 270
   Lys Asp Val Thr Ala Ala Val Leu Lys Asn Pro Ile Pro Val Leu Asn
   275 280 285
   Asn Ser Leu Pro Tyr Thr Ala Ile Ser Asn Ile Asn Gln Ile Ile Gln
   290 295 300
   Arg Lys Phe Leu Ser Glu Asn Ile Gln Glu Thr Ala Lys Leu Ile Asn
   305 310 315 320
   Met Ala Lys Lys Pro Val Ile Tyr Ala Gly Ala Gly Ile Leu Ser Asn
   325 330 335
   Leu Asn Gly Pro Thr Leu Leu Lys Glu Leu Ser Glu Arg Ser Gln Ile
   340 345 350
   Pro Val Thr Thr Thr Leu Gln Ala Leu Gly Ala Phe Asp Gln Gln Asp
   355 360 365
   Pro Lys Ser Leu Asp Met Leu Gly Met His Gly Ser Ala Val Ala Asn
   370 375 380
   Leu Ala Ile Gln Asn Ala Asp Leu Ile Ile Ala Leu Gly Gly Arg Phe
   385 390 395 400
   Asp Asp Arg Val Thr Gly Asn Ile Ser Lys Phe Ala Pro Glu Ala Arg
   405 410 415
   Lys Ala Ala Leu Glu Lys Arg Gly Gly Ile Val His Phe Glu Ile Ser
   420 425 430
   Pro Lys Asn Ile Asn Lys Val Val Glu Thr Gln Ile Ala Leu Glu Gly
   435 440 445
   Asp Val Thr Glu Asn Leu Glu Ile Leu Leu Pro Leu Ile Lys Ser Val
   450 455 460
   Asp Glu Arg Pro Glu Trp Phe Ala Gln Ile Asn Gln Trp Lys Lys Ala
   465 470 475 480
   Tyr Pro Tyr Glu Tyr Met Arg Glu Thr Pro Gly Ser Lys Ile Lys Pro
   485 490 495
   Gln Thr Val Ile Ser Arg Leu Ser Asp Ile Ala Asn Ser Thr Gly Lys
   500 505 510
   Lys Val Ile Val Thr Thr Gly Val Gly Gln His Gln Met Trp Thr Ala
   515 520 525
   Gln His Trp Thr Trp Arg Asn Pro Arg Thr Phe Ile Thr Ser Gly Gly
   530 535 540
   Leu Gly Thr Met Gly Tyr Gly Leu Pro Ala Ala Ile Gly Ala Gln Val
   545 550 555 560
   Ala Phe Pro Glu Ala Leu Val Ile Asp Ile Asp Gly Asp Ala Ser Phe
   565 570 575
   Asn Met Thr Leu Thr Glu Leu Ser Ser Ala Val Gln Ala Asn Thr Pro
                   580                 585                 590
   Ile Lys Ile Met Leu Leu Asn Asn Glu Glu Gln Gly Met Val Thr Gln
           595                 600                 605
   Trp Gln Thr Leu Phe Tyr Glu His Arg Tyr Ser His Thr His Gln Met
       610                 615                 620
   Asn Pro Asn Phe Gln Lys Leu Ala Glu Ala Met Gly Leu Lys Ala Met
   625                 630                 635                 640
   Lys Leu Lys Glu Tyr Glu Gln Ile Asp Ser Thr Leu Lys Glu Phe Leu
                   645                 650                 655
   Glu His Glu Gly Pro Val Leu Leu Glu Val Glu Ile Glu Lys Lys Val
               660                 665                 670
   Pro Val Leu Pro Met Val Pro Ala Gly Lys Gly Leu Asp Glu Phe Met
           675                 680                 685
   Tyr Phe Asp Pro Lys Thr Glu Lys Glu Gln Glu Lys Ile Arg Phe Glu
       690                 695                 700
   Arg Thr Glu Gly Lys His
   705                 710